(19) Europäisches Patentamt
European Patent Office
Office européen des brevets

(11) **EP 2 896 645 B1**

(12) **EUROPEAN PATENT SPECIFICATION**

(45) Date of publication and mention of the grant of the patent:
**11.12.2019 Bulletin 2019/50**

(21) Application number: **13837534.0**

(22) Date of filing: **20.08.2013**

(51) Int Cl.:
*C08J 3/24* (2006.01)   *B01J 20/26* (2006.01)
*B01J 20/30* (2006.01)   *C08F 220/06* (2006.01)
*A61L 15/60* (2006.01)   *A61L 15/24* (2006.01)

(86) International application number:
**PCT/JP2013/072206**

(87) International publication number:
**WO 2014/041968 (20.03.2014 Gazette 2014/12)**

(54) **METHOD FOR MANUFACTURING POLYACRYLIC ACID (POLYACRYLATE)-BASED WATER-ABSORBENT AGENT, AND WATER-ABSORBENT AGENT**

VERFAHREN ZUR HERSTELLUNG EINES WASSERABSORPTIONSMITTELS AUF BASIS VON POLYACRYLSÄULE (POLYACRYLAT) UND WASSERABSORPTIONSMITTEL

PROCÉDÉ DE FABRICATION D'AGENT ABSORBANT L'EAU À BASE D'ACIDE POLYACRYLIQUE (POLYACRYLATE) ET AGENT ABSORBANT L'EAU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **11.09.2012 JP 2012199958**

(43) Date of publication of application:
**22.07.2015 Bulletin 2015/30**

(73) Proprietor: **Nippon Shokubai Co., Ltd.**
**Osaka-shi, Osaka 541-0043 (JP)**

(72) Inventors:
• **KIMURA, Kazuki**
**Himeji-shi**
**Hyogo 671-1282 (JP)**
• **WATANABE, Yusuke**
**Himeji-shi**
**Hyogo 671-1282 (JP)**
• **MACHIDA, Sayaka**
**Himeji-shi**
**Hyogo 671-1282 (JP)**
• **FUJIMOTO, Taku**
**Himeji-shi**
**Hyogo 671-1282 (JP)**
• **KADONAGA, Kenji**
**Himeji-shi**
**Hyogo 671-1282 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 471 843     EP-A2- 1 191 051**
**WO-A1-2008/120742   WO-A1-2012/102406**
**JP-A- 2002 201 290   JP-A- 2004 352 776**
**JP-A- 2009 509 723**

• **DATABASE WPI Week 201175 Thomson Scientific, London, GB; AN 2011-N64801 XP002753405, & WO 2011/136301 A1 (NIPPON SHOKUBAI CO LTD) 3 November 2011 (2011-11-03)**

**Description**

Technical Field

The present invention relates to a method of producing a polyacrylic acid (salt)-based water absorbing agent and to the water absorbing agent. More specifically, the present invention relates to (i) a method of producing a water absorbing agent to be used for sanitary materials such as disposable diapers, sanitary napkins, and incontinence pads and (ii) the water absorbing agent obtained by the method.

Background Art

Currently, an absorbent body, which is constituted by hydrophilic tissue such as pulp and by a water absorbing agent mainly made from acrylic acid (salt) or the like, is put to widespread use in sanitary materials such as disposable diapers, sanitary napkins and incontinence pads. The absorbent body is used for the purpose of absorbing bodily fluids. In recent years, these sanitary materials such as disposable diapers and sanitary napkins have been enhanced in functionality and made thinner. Accordingly, there have been increases in (i) the amount of water absorbing agent used for a piece of sanitary material and (ii) a water absorbing agent content in an entire absorbent body constituted by a water absorbing agent, hydrophilic tissue, and the like. That is, research has been conducted on reduction in the thickness of a sanitary material without reducing the amount of water absorption by increasing the ratio of water absorbing agent contained in an absorbent body through (i) using a smaller amount of hydrophilic tissue having small bulk specific gravity and (ii) using a larger amount of water absorbing agent having excellent water absorbency and large bulk specific gravity.

Such a sanitary material, in which a water absorbing agent content is increased by decreasing the ratio of hydrophilic tissue, is preferable from the viewpoint of simply holding a liquid. However, such a sanitary material is rather problematic if distribution and spreading of a liquid when the sanitary material is actually used are taken into consideration.

When absorbing water, a large amount of water absorbing agent per unit volume turns into a softer gel-like form. This induces such a phenomenon as gel blocking which causes considerable reduction in diffusibility of a liquid in a sanitary material. As a result, part of the water absorbing agent, which part is distant from a central area of the sanitary material and is therefore difficult for the liquid to reach, does not effectively function. This prevents the effect of increasing the water absorbing agent content from being sufficiently exerted, and therefore causes an absorbing ability of the sanitary material in actual use to be much lower than a theoretical level.

In order to avoid the problem and maintain absorption property of an absorbent body, a range of the ratio between hydrophilic tissue and a water absorbing agent is inevitably limited. This puts limitations on thinning of sanitary materials.

Examples of an index by which an improvement of gel blocking in a sanitary material is evaluated encompass (i) absorbency against pressure (AAP) or performance under pressure (PUP) and (ii) saline flow conductivity (hereinafter abbreviated as "SFC"; see Patent Literature 1), and the like.

Examples of a well-known technique for improving gel blocking encompass a technique in which crosslinking densities inside and outside of a water absorbing agent are changed by surface treatment (see Patent Literatures 2 to 21). There are also known attempts to improve water absorbing ability, particularly liquid diffusibility, by combining surface treatment with, as a liquid permeability enhancer, (i) inorganic fine particles, (ii) inorganic compounds such as polyvalent metal salt, or (iii) cationic polymer compounds (see Patent Literatures 22 to 35). Furthermore, a technique for controlling a reaction environment of surface crosslinking treatment is also known (see Patent Literatures 36 to 39).

However, while the aforementioned well-known methods can prevent gel blocking, the methods also pose, in actual production of a water absorbing agent, the following problems: (i) Liquid diffusibility, particularly saline flow conductivity (hereinafter also referred to as "SFC") and gel bed permeability (hereinafter also referred to as "GBP"), in a sanitary material does not attain desired performance. (ii) Even though SFC and GBP are sufficient, a surface crosslinking reaction at a low speed causes much time for developing the reaction to the extent that absorption capacity (CRC) reaches a desired level. This causes the impossibility of producing a water absorbing agent with high productivity and further causes a residual of a used surface crosslinking agent to remain on the surface of the water absorbent resin.

That is, a first objective is to enhance productivity, particularly industrial-scale productivity, involved in the above enhanced functionality.

Production of a water absorbent resin includes a lot of steps such as a neutralization step, a polymerization step, a drying step, a pulverization step, a classification step, a fine powder recycling step, a surface crosslinking step, a granulation step, and a modifier/additive step. In the industrial production (especially in production based on aqueous polymerization), these steps are connected with each other to realize a continuous production.

However, in most of the recent techniques, like Patent Literatures 1 to 39 listed below, for realizing the enhanced functionality (e.g. high liquid permeability), the surface crosslinking step can be a rate-determining step. For this reason, importance has been placed on enhancement of productivity in the surface crosslinking step (shortening of a reaction time). Further, in a huge-scale continuous production (e.g. not less than 100 kg/hr), a long reaction time increases a mechanical load in a heat reactor and a load caused by self weight of a water absorbent resin on a bottom surface of the reactor, thus increasing the amount of fine powder from the water absorbent resin.

Some methods for enhancing productivity (shortening a reaction time) in the surface crosslinking step include a method of decreasing a neutralization rate (Patent Literature 40) and a method of increasing a reaction temperature. These methods, however, cause decreased functionality (physical property) and coloring of a resultant water absorbing agent, a restriction on the reaction temperature in order to avoid thermal deterioration (decrease in heat resistance) of a water absorbent resin, a limitation of heating capability of an apparatus, and other problems. Other method for shortening a reaction time includes a method of increasing the amount of surface crosslinking agent. This, however, causes an increased cost for raw materials and a residual surface crosslinking agent.

Further, a second objective is to solve a problem of a residual surface crosslinking agent, i.e. the problem that a residual of a surface crosslinking agent used in the surface crosslinking step and/or a byproduct derived from the surface crosslinking agent is generated or remained. Especially the recent thinning of sanitary materials is feasible with increases in absolute amount and density of a water absorbing agent per sheet. It is therefore important to minimize an unreacted material and a byproduct even when raw materials having a high degree of safety are used. For this reason, there has been a demand for a method for effectively reducing such an unreacted material or a byproduct.

Examples of a method for reducing an unreacted material derived from a surface crosslinking agent include: a method of, when a surface crosslinking agent is an epoxy compound, adding an additive selected from the group consisting of saturated inorganic acids and saturated organic acids (Patent Literature 41); a method of, when a surface crosslinking agent is an epoxy compound, adding a nucleophilic agent such as water under a particular condition or performing cleaning (Patent Literature 42); a method of, when a surface crosslinking agent is a crosslinking agent selected from polyhydric alcohols, alkylene carbonates, oxazolidinone compounds, and (polyhydric) oxetane compounds, subjecting a heat-treated water absorbent resin powder to cooling under an airflow and, simultaneously, removing at least part of a residual surface crosslinking agent from the water absorbent resin powder by an airflow (Patent Literature 43); a method of, when a surface crosslinking agent is an alcohol-based compound (except for ethylene glycol) selected from amino alcohols, alkylene carbonates, and polyhydric alcohols, exposing a water absorbent resin in course of heat treatment and/or a heat-treated water absorbent resin to an airflow of an atmospheric temperature of not lower than 60 °C (Patent Literature 44); and the like.

However, these methods require the additional step(s) and thus give rise to problems such as decrease in productivity and decrease in physical property (gel blocking property) of a water absorbing agent. In addition, these methods can be less effective depending on a surface crosslinking agent to be used. Especially, in a case where the surface crosslinking agent to be used is a polyhydric alcohol compound or an amino alcohol compound, which are not so high in reactivity with a water absorbent resin and in a case where a surface crosslinking agent that generates, as a byproduct, a polyhydric alcohol compound or an amino alcohol compound is used, there is no method for effectively reducing an unreacted material.

Moreover, a residual surface crosslinking agent can cause not only the problem from a safety standpoint but also decrease in Anti-Caking property at the moisture absorption and decrease in powder fluidity. For example, in a case where an alkylene carbonate compound is used, the alkylene carbonate compound can generate, as a byproduct, a polyhydric alcohol (particularly ethylene glycol) that is a degradation product of the alkylene carbonate compound. In order to solve this problem, the methods disclosed in Patent Literatures 45 and 46 have been proposed.

Citation List

Patent Literatures

[Patent Literature 1]
Pamphlet of International Publication No. WO 95/26209
[Patent Literature 2]
Specification of U.S. Patent No. 6297319
[Patent Literature 3]
Specification of U.S. Patent No. 6372852
[Patent Literature 4]
Specification of U.S. Patent No. 6265488
[Patent Literature 5]
Specification of U.S. Patent No. 6809158
[Patent Literature 6]

Specification of U.S. Patent No. 4734478
[Patent Literature 7]
Specification of U.S. Patent No. 4755562
[Patent Literature 8]
Specification of U.S. Patent No. 4824901
[Patent Literature 9]
Specification of U.S. Patent No. 6239230
[Patent Literature 10]
Specification of U.S. Patent No. 6559239
[Patent Literature 11]
Specification of U.S. Patent No. 6472478
[Patent Literature 12]
Specification of U.S. Patent No. 6657015
[Patent Literature 13]
Specification of U.S. Patent No. 5672633
[Patent Literature 14]
Specification of European Patent Application Publication No. 0940149
[Patent Literature 15]
Pamphlet of International Publication No. WO 2006/033477
[Patent Literature 16]
Specification of U.S. Patent No. 7201941
[Patent Literature 17]
Specification of U.S. Patent No. 4783510
[Patent Literature 18]
Specification of European Patent No. 1824910
[Patent Literature 19]
Pamphlet of International Publication No. WO 2002/100451
[Patent Literature 20]
Specification of U.S. Patent No. 5610208
[Patent Literature 21]
Pamphlet of International Publication No. WO 92/000108
[Patent Literature 22]
Pamphlet of International Publication No. WO 98/49221
[Patent Literature 23]
Pamphlet of International Publication No. WO 00/53644
[Patent Literature 24]
Pamphlet of International Publication No. WO 00/53664
[Patent Literature 25]
Pamphlet of International Publication No. WO 01/074913
[Patent Literature 26]
Pamphlet of International Publication No. WO 2002/020068
[Patent Literature 27]
Pamphlet of International Publication No. WO 2002/022717
[Patent Literature 28]
Pamphlet of International Publication No. WO 2005/080479
[Patent Literature 29]
Pamphlet of International Publication No. WO 2007/065834
[Patent Literature 30]
Pamphlet of International Publication No. WO 2008/092842
[Patent Literature 31]
Pamphlet of International Publication No. WO 2008/092843
[Patent Literature 32]
Pamphlet of International Publication No. WO 2008/110524
[Patent Literature 33]
Pamphlet of International Publication No. WO 2009/080611
[Patent Literature 34]
Japanese Examined Patent Application Publication, Tokukouhei, No. 4-46617
[Patent Literature 35]

Pamphlet of International Publication No. WO 00/46260
[Patent Literature 36]
Specification of European Patent No. 1191051
[Patent Literature 37]
Pamphlet of International Publication No. WO 2011/117263
[Patent Literature 38]
Pamphlet of International Publication No. WO 09/125849
[Patent Literature 39]
Korean Patent No. 2011/0049072A
[Patent Literature 40]
Pamphlet of International Publication No. WO 2006/042704
[Patent Literature 41]
Specification of European Patent No. 0668080
[Patent Literature 42]
Pamphlet of International Publication No. WO 97/0003114
[Patent Literature 43]
Specification of U.S. Patent No. 7378453
[Patent Literature 44]
Pamphlet of International Publication No. WO 2006/033477
[Patent Literature 45]
Pamphlet of International Publication No. WO 2012/102406
[Patent Literature 46]
Pamphlet of International Publication No. WO 2012/102407

Non-Patent Literature

[Non-Patent Literature 1]
Modern Superabsorbent Polymer Technology (1998) (particularly, p.39-44, p.97-103, p.197-199, etc.)

Summary of Invention

Technical Problem

An object of the present invention is to provide a production method that enables maintaining or enhancing productivity of a water absorbing agent, reducing a residual surface crosslinking agent, and increasing Anti-Caking property of a water absorbing agent, in production of water absorbing agents having a high functionality (especially high liquid permeability and high Anti-Caking property) in Patent Literatures 1 to 39 listed above.
That is, the present invention relates to a method for producing a water absorbing agent with stability and high productivity in actual production, the method solving the aforementioned problems, wherein the water absorbing agent is less likely to cause gel blocking, is suitable for use in thin sanitary materials and absorbent articles which are high in water absorbing agent content, and is excellent in liquid diffusibility (e.g. SFC).

Solution to Problem

In order to solve the above problems, the inventors of the present invention made a diligent study and accomplished the present invention by finding that in a method for producing a water absorbing agent, in which a polyacrylic acid (salt)-based water absorbent resin obtained by polymerization of a hydrophilic unsaturated monomer is subjected to a surface crosslinking agent addition step of adding a surface crosslinking agent and/or a surface crosslinking agent solution and a surface crosslinking step, wherein (i) in the surface crosslinking step performed after the surface crosslinking agent addition step, a maximum temperature in an atmosphere within a heating section of a heating apparatus used in the surface crosslinking step is in a range from 100 °C to 300 °C, and a minimum dew point in the atmosphere is lower than 45 °C, and wherein (ii) a liquid permeability enhancer addition step of adding a liquid permeability enhancer is performed simultaneously with the surface crosslinking agent addition step and/or after the surface crosslinking step, whereby a water absorbing agent having a high liquid permeability and, an excellent antiblocking property (Anti-Caking property) after moisture absorption, as an additional effect, is obtained with high productivity. Furthermore, the inventors of the present invention also found surprisingly that (a) particles obtained by drying and pulverization of fine particles removed by the classification step and then granulated are recycled for a water absorbent resin to be subjected to the surface crosslinking step, thereby further enhancing productivity, and that (b) containing fine particles which are removed from the water absorbing agent undergoing a second classification step performed after the surface crosslinking step provides a noticeable enhancement in productivity.

That is, the present invention is a method for producing a polyacrylic acid (salt)-based water absorbing agent, including:

a surface crosslinking agent addition step of adding a surface crosslinking agent to a water absorbent resin powder; a surface crosslinking step; and

a liquid permeability enhancer addition step of adding a liquid permeability enhancer that is selected from insoluble fine particulate compounds and polyvalent cationic compounds, the liquid permeability enhancer addition step being performed simultaneously with the surface crosslinking agent addition step and/or after the surface crosslinking step, wherein a maximum temperature in an atmosphere within a heating section of a heating apparatus used in the surface crosslinking step is in a range from 100 °C to 300 °C, and a minimum dew point in the atmosphere is lower than 45 °C, and (1) an airflow rate in the heating apparatus is more than 0 but not more than 10,000 Nm3 /hr or (2) an airflow in the heating apparatus has a ratio of gas relative to an amount of the water absorbent resin powder of not more than 3000 Nm3 /ton, and the dew point is adjusted by the airflow using steam, dry air, nitrogen, helium, argon, dried air, and/or water vapor generated from water contained in the water absorbent resin powder.

Advantageous Effects of Invention

According to a method for producing a water absorbing agent in accordance with the present invention, it is possible to efficiently produce a water absorbing agent with ease, whereby a residual surface crosslinking agent in the water absorbing agent is reduced, the water absorbing agent has a high liquid permeability, and a time for the surface crosslinking step involving a high-temperature heat treatment, which step tends to be a bottleneck on an industrial scale, is shortened.

Description of Embodiments

Hereinafter, a method for producing a polyacrylic acid (salt)-based water absorbing agent according to the present invention will be described in detail. In the present invention, the terms "weight" and "mass", "wt%" and "mass%", and "parts by weight" and "parts by mass" are synonymous with each other correspondingly, and the terms "mass", "mass%", and "parts by mass" are used herein for consistency.

[1] Definitions of terms

(1-1) Water absorbing agent

The term "water absorbing agent" as used herein means a gelatinizer for an aqueous liquid, which gelatinizer contains a water absorbent resin of not less than 70 mass%, preferably not less than 85 mass%, obtained by subjecting a water absorbent resin to a surface crosslinking step and a step of adding a liquid permeability enhancer (hereinafter also referred to as "liquid permeability enhancer addition step"). Not only a surface crosslinking agent and the liquid permeability enhancer, but also a chelating agent, a reducing agent, an antioxidant, an anti-coloring agent, etc., each in an amount of 0 to 10 mass%, preferably 0.1 to 1 mass%, relative to the amount of water absorbent resin, may be added to or contained in the water absorbent resin.

(1-2) Surface-crosslinked water absorbent resin

The term "surface-crosslinked water absorbent resin" as used herein means a gelatinizer for an aqueous solution, which gelatinizer is obtained by subjecting a water absorbent resin to the surface crosslinking step. A product obtained by subjecting a water absorbent resin to the surface crosslinking step after the surface crosslinking agent addition step and the liquid permeability enhancer addition step is also referred to as "surface-crosslinked water absorbent resin."

(1-3) "Polyacrylic acid (salt)-based water absorbent resin"

The term "water absorbent resin" as used herein means a water-swellable or water-insoluble polymer gelatinizer. Note that the "water-swellable" property is a property of having CRC (centrifuge retention capacity), of not less than 5 [g/g], where the CRC is defined in ERT 441.2-02, and the "water-insoluble" property is a property of having Extr (water soluble component) of 0 to 50 mass%, where the Extr is defined in ERT 470.2-02.

The water absorbent resin is not limited to a resin in which the water absorbent resin is totally a polymer (100 mass%), and therefore can be the one that contains an additive or the like, provided that the above "water-swellable" and "water-insoluble" properties are ensured. A water absorbent resin composition containing a small amount of additive is also referred collectively to as the water absorbent resin in accordance with the present invention. Moreover, the form of the water absorbent resin is not particularly limited. Examples of the form of the water absorbent resin include a sheet form, a fiber form, a film form, a gel form, a powder form, and the like. The water absorbent resin is preferably in the form of powder, particularly preferably in the form of powder having the particle size and moisture content, both of which will be described later. Such a water absorbent resin may be referred to as a water absorbent resin powder.

The term "polyacrylic acid (salt)-based water absorbent resin" as used herein means a polymer that optionally has a graft component and contains, as main components, recurring units constituted by an acrylic acid and/or a salt thereof (hereinafter referred to as acrylic acid (salt)).

More specifically, the "polyacrylic acid (salt)-based water absorbent resin" as used in the present invention is a polymer in which acrylic acid (salt) accounts for 50 mol% to 100 mol% in the total monomer content (except a crosslinking agent) to be polymerized, preferably a water absorbent resin in which acrylic acid (salt) accounts for 70 mol% to 100 mol% in the total monomer content, more preferably a water absorbent resin in which acrylic acid (salt) accounts for 90 mol% to 100 mol% in the total monomer content, and particularly preferably a water absorbent resin in which acrylic acid (salt) accounts for substantially 100 mol% in the total monomer content. Moreover, in the present invention, a polyacrylate (neutralized) polymer is also referred collectively to as "polyacrylic acid (salt)-based water absorbent resin".

(1-4) "EDANA" and "ERT"

The term "EDANA" stands for European Disposables and Nonwovens Associations. The term "ERT" stands for EDANA Recommended Test Methods, which is the European-standard (actually the global-standard) method of measuring water absorbent resins. The ERT is a method of measuring the physical properties of water absorbent resins, and unless otherwise specified, the measurement of the physical properties of the water absorbent resin herein is carried out in conformity with a master copy of the ERT (Known Literature: 2002 revised version).

(a) "CRC" (ERT441.2-02)

The term "CRC" stands for a centrifuge retention capacity and means absorption capacity without load (hereinafter may be referred simply to as "absorption capacity"). Specifically, the "CRC" means absorption capacity (Unit: [g/g]) observed when 0.200 g of water absorbent resin wrapped in unwoven cloth is allowed to freely swell in a 0.9 mass% of sodium chloride aqueous solution(physiological saline) without load for 30 minutes and then drained by a centrifugal machine.

(b) "AAP" (ERT442.2-02)

The term "AAP" stands for Absorption Against Pressure and means absorption capacity under load. Specifically, the "AAP" means absorption capacity (Unit: [g/g]) observed after 0.900 g of water absorbent resin is allowed to swell in a 0.9 mass% of sodium chloride aqueous solution (physiological saline) under a load for 1 hour. It should be noted that the measurement of the AAP herein is different from the ERT 442.2-02 in that the AAP is measured under a load of 4.83 kPa (0.7 psi).

(c) "PSD" (ERT420.2-02)

The term "PSD" stands for Particle Size Distribution and means a particle size distribution measured by sieve classification. It should be noted that the mass average particle diameter (D50) and the distribution of particle diameter are measured by the method as set forth in "Average Particle Diameter and Distribution of Particle Diameter" of U.S. Patent No. 2006/204755.

(1-6) "Liquid permeability"

The degree of flowing of a liquid between particles of swollen water absorbent resin under load or without load is referred to as "liquid permeability". The "liquid permeability" is measured typically as SFC (Saline Flow Conductivity) or GBP (Gel Bed Permeability).

"SFC (Saline Flow Conductivity)" is liquid permeability of 0.9 g of water absorbent resin for a 0.69 mass% of sodium chloride aqueous solution under load of 2.07 kPa, and is measured according to the SFC test method disclosed in the specification of U.S. patent No. 5669894. Moreover, "GBP" is liquid permeability of a water absorbent resin for a 0.69 mass% of sodium chloride aqueous solution wherein the water absorbent resin is under load or allowed to freely swell, and is measured according to the GBP test method disclosed in the International Publication No. WO 2005/016393.

(1-7) Others

The expression "X to Y" for expression of a range herein means "not less than X and not more than Y" in which both X and Y are inclusive. Moreover, the weight unit "t (ton)" means "Metric ton". Further, unless otherwise specified, "ppm" means "ppm by mass". Further, the wording "... acid (salt)" means "... acid and/or salt thereof". The wording "(meth)acrylic" means "acrylic and/or methacrylic". Besides, unless otherwise specified, physical properties and the like are measured at room temperature (20 to 25 C°) at a relative humidity of 40 to 50 % RH.

[2] A process for production of polyacrylic acid (salt)-based water absorbent resin powder

(2-1) Step of preparing acrylic acid (salt)-based monomer aqueous solution

As used herein, the term "acrylic acid (salt)-based monomer aqueous solution" is an aqueous solution of a monomer(s) (hereinafter referred to as "monomer aqueous solution") which are mainly acrylic acid (salt). The acrylic acid (salt)-based monomer aqueous solution may contain, if necessary, constituent components of the water absorbent resin powder, such as a crosslinking agent, a graft component, a minute component (chelating agent, surfactant, a dispersing agent, or the like). It is possible to perform the polymerization with the acrylic acid (salt)-based monomer aqueous solution as such and a polymerization initiator added thereto.

The acrylic acid (salt) may be not neutralized or may be a salt (fully neutralized or partially neutralized). Moreover, the monomer aqueous solution may exceed its saturation concentration. The acrylic acid (salt)-based monomer aqueous solution in the present invention also encompasses a supersaturated aqueous solution or a slurry aqueous solution (aqueous dispersion solution) of the acrylic acid (salt). From the viewpoint of physical properties of the resultant water absorbent resin powder, it is preferable that the acrylic acid (salt)-based monomer aqueous solution not saturated is used.

A solvent for the monomer(s) is preferably water. In a case where the solvent for the monomer(s) is water, an acrylic acid (salt)-based monomer is dealt as an aqueous solution. The term "aqueous solution" as used herein is not limited to a solution containing water of 100 mass% as a solvent and may contain, as the solvent, a combination of water and a water-soluble organic solvent (e.g. alcohol etc.) of 0-30 mass%, preferably 0 to 5 mass%. These are dealt herein as "aqueous solutions."

As used herein, the term "partly-prepared acrylic acid (salt)-based monomer aqueous solution" refers to an aqueous solution of acrylic acid (salt), which is to be prepared as a monomer aqueous solution whose main component is acrylic acid and/or its salt, but to which not all constituent components have been added. Specifically, the partly-prepared acrylic acid (salt)-based monomer aqueous solution includes an acrylic acid aqueous solution and a fully or partly neutralized acrylic acid salt aqueous solution.

The partly-prepared acrylic acid (salt)-based monomer aqueous solution is to be further neutralized, mixed with water as a solvent, or mixed with the minute component(s), thereby being prepared as the fully prepared acrylic acid (salt)-based monomer aqueous solution. It should be noted that the fully prepared acrylic acid (salt)-based monomer aqueous solution, when it is in such a state that it has not been introduced into a polymerizer or has been introduced into the polymerizer but whose polymerization has not been started yet, is referred to as "pre-polymerization fully prepared acrylic acid (salt)-based monomer aqueous solution".

(Monomer)

The acrylic acid (salt)-based monomer according to the present invention is not particularly limited, provided that a water absorbent resin can be produced therefrom by polymerization, and examples thereof encompasses: anionic unsaturated monomers and salt thereof such as (meth)acrylic acid, (anhydrous)maleic acid, itaconic acid, cinnamic acid, vinylsulfonic acid, allyltoluenesulfonic acid, vinyltoluene sulfonic acid, styrene sulfonic acid, 2-(meth) acrylamide-2-methylpropanesulfonic acid, 2-(meth) acryloyl ethanesulfonic acid, 2-(meth) acryloyl propanesulfonic acid, and 2-hydroxyethyl (meth) acryloyl phosphate; mercapto group-containing unsaturated monomers; phenolic hydroxide group-containing unsaturated monomers; amide group-containing unsaturated monomers such as (meth) acrylamide, N-ethyl (meth) acrylamide, N,N-dimethyl (meth) acrylamide; amino group-containing unsaturated monomers such as N,N-dimethylamino ethyl (meth) acrylate, N,N-dimethylaminopropyl (meth) acrylate, and N,N-dimethylaminopropyl (meth) acrylamide; and the other monomers.

The content (used amount) of the acrylic acid (salt)-based monomer is normally not less than 50 mol %, preferably not less than 70 mol %, more preferably not less than 80 mol %, still more preferably not less than 90 mol %, and particularly preferably not less than 95 mol % (upper limit is 100 mol %), relative to a total monomer content (excluding an internal crosslinking agent). It should be noted that the polyacrylic acid (salt) according to the present invention, which is not limited to a non-neutralized one (0 mol % neutralization rate), is defined as encompassing a partially neutralized or fully neutralized one (100 mol % neutralization rate).

A neutralization rate of the acrylic acid (salt)-based monomer or the water-containing gel-like crosslinked polymer after the polymerization according to the present invention is not particularly limited to a specific neutralization rate, but is preferably in a range from 40 mol% to 90 mol%, more preferably in a range from 50 mol% to 80 mol%, and still more preferably in a range from 60 mol% to 74 mol%, from the viewpoint of physical properties of a resultant water absorbent resin powder or reactivity of the surface crosslinking agent.

It is preferable that the neutralization rate is within these ranges, because a low neutralization rate tends to lower a water absorbing speed (for example, FSR) of a resultant water absorbent resin, and a high neutralization rate tends to lower reactivity of the polyacrylic acid (salt)-based water absorbent resin powder with a surface crosslinking agent, particularly with a dehydration reaction surface crosslinking agent described later, still particularly with alkylene carbonate, thereby resulting in low yield of a water absorbent resin or low liquid permeability (for example, SFC) and low absorption capacity under load (for example, AAP or PUP) of the water absorbent resin.

The acrylic acid (salt)-based monomer or the water-containing gel-like crosslinked polymer may be partly or totally salt from the viewpoint of the absorption capacity without load (CRC) and the absorption capacity under load (AAP) of a water absorbing agent obtained as a final product. The acrylic acid (salt)-based monomer or the water-containing gel-like crosslinked polymer is preferably monovalent salts such as alkaline metal salt (sodium salt, lithium salt, and potassium salt), ammonium salt, and amines. Among them, the acrylic acid (salt)-based monomer or the water-containing gel-like crosslinked polymer is more preferably alkaline metal salt, still more preferably sodium salt and/or potassium salt, and particularly preferably sodium salt from the viewpoint of cost and physical property.

(Polymerization inhibitor)

The acrylic acid (salt)-based monomer according to the present invention contains a polymerization inhibitor. Although the polymerization inhibitor is not particularly limited, examples of the polymerization inhibitor encompass N-oxyl compounds, manganese compounds, substituted phenol compounds, and the like, which are disclosed in International Publication No. WO 2008/096713. Among these compounds, the substituted phenol compounds are preferable, and among the substituted phenol compounds, methoxy phenols are particularly preferable.

Examples of the methoxy phenols encompass o, m, p-methoxy phenol, methoxy phenols substituted with one or more substituents such as a methyl group, a t-buthyl group, or a hydroxyl group, and the like. In the present invention, p-methoxy phenol is particularly preferable.

The polymerization inhibitor content in the acrylic acid (salt)-based monomer is preferably in a range of 10 ppm to 200 ppm, more preferably in a range of 5 ppm to 160 ppm, 10 ppm to 160 ppm, 10 ppm to 100 ppm, 10 ppm to 80 ppm, in this order, and most preferably in a range of 10 ppm to 70 ppm, relative to a total amount of the acrylic acid (salt)-based monomer. If the polymerization inhibitor content of more than 200 ppm, deterioration in color tone (yellowing, yellow color change) may occur on the resultant water absorbing agent. If the polymerization inhibitor content is less than 5 ppm, that is, if the polymerization inhibitor is removed due to refining such as distillation or the like, unintentional polymerization may take place.

(Internal crosslinking agent)

In the present invention, at the polymerization, an internal crosslinking agent is used if necessary. The internal crosslinking agent is not particularly limited and can be a publicly known internal crosslinking agent. Examples of the internal crosslinking agents encompass: N,N'-methylenebis (meth) acrylamide, (poly)ethylene glycol di(meth) acrylate, (poly)propyleneglycol di(meth) acrylate, trimethylolpropane tri(meth)acrylate, glycerine tri(meth)acrylate, glycerine acrylate methacrylate, ethyleneoxide modified trimethylol propane tri(meth)acrylate, pentaerythritol hexa(meth)acrylate, triallyl cyanurate, triallyl isocyanurate, triallyl phosphate, triallylamine, poly(meth)allyloxy alkanes, (poly) ethylene glycol diglycidyl ether, glycerol diglycidyl ether, ethylene glycol, polyethylene glycol, propylene glycol, glycerin, 1,4-butanediol, pentaerythritol, ethylenediamine, ethylene carbonate, propylene carbonate, polyethyleneimine, glycidyl (meth) acrylate, and the like. In consideration of reactivity, one or more kinds of compounds among them can be used. Especially, it is preferable to use a compound having two or more polymerizable unsaturated groups.

Further, in a case where two or more internal crosslinking agents are used in combination, an internal crosslinkage structure can be changed by changing reactivities of functional groups of the internal crosslinking agents. As such, it is preferable that internal crosslinking agents having different functional groups are selected for use in combination from among amide compounds, (meth) acrylate compounds, allylic compounds, amine compounds, imine compounds, alcohol compounds, carbonate compounds, and glycidyl compounds.

A used amount of the internal crosslinking agent can be determined as appropriate, depending on desired physical properties of the water absorbing agent. However, the used amount of the internal crosslinking agent is preferably in a range from 0.001 mol % to 5 mol %, more preferably in a range from 0.005 mol % to 2 mol %, and still more preferably in a range from 0.01 mol % to 1 mol %, relative to a total amount of the acrylic acid (salt)-based monomer(s). If two or more internal crosslinking agents are used in combination, the used amount of each of the internal crosslinking agents is preferably in a range from 0.001 mol % to 5 mol %, more preferably in a range from 0.005 mol % to 2 mol %, and still more preferably in a range from 0.01 mol % to 1 mol %, relative to a total amount of the acrylic acid (salt)-based monomer(s).

If the used amount of the internal crosslinking agent (or a total amount of the two or more internal crosslinking agents used in combination) is less than 0.001 mol %, a resultant water absorbing agent has a high water soluble component, thereby resulting in a risk of failing to ensure a sufficient absorbing amount under load. On the other hand, if the used amount of the internal crosslinking agent exceeds 5 mol %, this would possibly result in a high crosslinking density of a resultant water absorbing agent, thereby resulting in an insufficient absorbing amount of the resultant water absorbing agent. It should be noted that the internal crosslinking agent(s) may be added in whole to the pre-polymerization fully prepared acrylic acid (salt)-based monomer aqueous solution, or a portion thereof may be added after the polymerization is initiated.

(Dispersing agent)

The dispersing agent usable in the present invention is not particularly limited, but is preferably a water absorbent polymer dispersing agent, a hydrophilic polymer dispersing agent that is water absorbable, or a water-soluble polymer dispersing agent, and more preferably the water-soluble polymer dispersing agent. A weight average molecular weight of the dispersing agent is determined as appropriate depending on the type of the dispersing agent. However, the weight average molecular weight of the dispersing agent is preferably in a range from 500 to 10,000,000, more preferably in a range from 5,000 to 5,000,000, and particularly preferably in a range from 10,000 to 3,000,000.

The dispersing agent is not limited to a specific kind of dispersing agent. Examples of the dispersing agent encompass hydrophilic polymers such as starch, a starch derivative, cellulose, cellulose derivative, polyvinyl alcohol (PVA), carboxymethyl cellulose (sodium), hydroxyethyl cellulose, polyacrylic acid (salt), and crosslinked polyacrylic acid (salt). Among them, a water-soluble polymer dispersing agent selected from starch, cellulose, and PVA is preferable from the viewpoint of not impairing hydrophilicity of the water absorbing agent of the present invention.

A used amount of the dispersing agent is preferably in a range from 0 part by mass to 50 parts by mass, more preferably in a range from 0.01 parts by mass to 20 parts by mass, still more preferably in a range from 0.05 parts by mass to 10 parts by mass, and specifically preferably in a range from 0.1 parts by mass to 5 parts by mass, relative to 100 parts by mass of the acrylic acid (salt)-based monomer. The use of more than 50 parts by mass of the dispersing agent may decrease absorption property of the water absorbing agent.

(2-2) Polymerization step

(Polymerization method)

A polymerization method for obtaining a water absorbent resin powder according to the present invention is exemplified by spraying polymerization, droplet polymerization, bulk polymerization, precipitation polymerization, aqueous polymerization, reverse-phase suspension polymerization, and other polymerizations. In order to achieve the object of the present invention, it is preferable to employ aqueous polymerization or reverse-phase suspension polymerization, each of which is carried out by using an aqueous solution of monomers.

The aqueous polymerization is a method of polymerizing a monomer aqueous solution without using a dispersion solvent. Such aqueous polymerization is any of the polymerization methods disclosed in, for example, U.S. Patent No. 4625001, U.S. Patent No. 4873299, U.S. Patent No. 4286082, U.S. Patent No. 4973632, U.S. Patent No. 4985518, U.S. Patent No. 5124416, U.S. Patent No. 5250640, U.S. Patent No. 5264495, U.S. Patent No. 5145906, U.S. Patent No. 5380808, and European Patent No. 0811636, European Patent No. 0955086, European Patent No. 0922717, and others.

The reverse-phase suspension polymerization is a method of carrying out polymerization by suspending a monomer aqueous solution in a hydrophobic organic solvent. Such reverse-phase suspension polymerization is any of the polymerization methods disclosed in, for example, U.S. Patent No. 4093776, U.S. Patent No. 4367323, U.S. patent No. 4446261, U.S. Patent No. 4683274, U.S. Patent No. 5244735, and others. The monomers, the polymerization initiators, etc. disclosed in these patent literatures are applicable to the reverse-phase suspension polymerization in the present invention.

The concentration of the monomer aqueous solution in the polymerization is not particularly limited, but is preferably in a range of 20 mass% to the saturated concentration, more preferably in a range from 25 mass% to 80 mass%, and still more preferably in a range from 30 mass% to 70 mass%. The monomer concentration of less than 20 mass% may decrease productivity. It should be noted that polymerization with a monomer slurry (aqueous dispersion of acrylate) results in polymers with poor properties. Thus, the polymerization is preferably carried out with a monomer concentration not more than the saturated concentration (see Japanese Patent Application Publication, Tokukaihei, No. 1-318021).

In order to promote the polymerization and to thereby improve the physical properties of a water absorbing agent, the step of degassing the dissolved oxygen (for example, the step of exchanging the dissolved oxygen with inert gas) may be provided if necessary during the polymerization. In addition, for the purpose of increasing the water absorbing speed of a water absorbing agent, increasing a surface area of the water absorbing agent, increasing drying speed of the water absorbing agent, or the like, air bubbles (particularly inert gas) or various kinds of foaming agents (for example, organic or inorganic carbonate, an azo compound, a urea compound) may be contained during the polymerization so that foams are formed so as to increase to, for example, 1.001 to 10 times by volume during the polymerization and during the drying.

Further, in order to promote foaming during the polymerization and to improve handling property such as fluidity of a resultant polymerized gel, a surfactant, which is different from another surfactant described later, may be added to a monomer aqueous solution before the polymerization step and/or during the polymerization step. As the surfactant used for this purpose, the surfactant described in paragraphs [0115] to [0123] of the pamphlet of International Publication No. WO 2011/078298 can be employed.

The polymerization in the present invention can be carried out under any of normal atmospheric pressure, reduced pressure, and increased pressure. Preferably, the polymerization is carried out under the normal atmospheric pressure (101.3 kPa (1 atmospheric pressure)) (or under an atmospheric pressure close to the normal atmospheric pressure (normal atmospheric pressure $\pm$ 10 %)). Depending upon the type of polymerization initiator to be used, the temperature at the initiation of polymerization is preferably in a range from 15 °C to 130 °C and more preferably in a range from 20 °C to 120 °C.

(Polymerization initiator)

A polymerization initiator used in the present invention is selected appropriately depending on a polymerization scheme, and is not particularly limited. Examples of the polymerization initiator include photodegradable polymerization initiators, pyrolytic polymerization initiators, redox polymerization initiators, and the like. With any of these polymerization initiators, polymerization in the present invention is initiated.

Examples of the photodegradable polymerization initiators encompass benzoin derivatives, benzyl derivatives, acetophenone derivatives, benzophenone derivatives, azo compounds.

Examples of the pyrolytic polymerization initiators encompass: persulfates such as sodium persulfate, potassium persulfate, and ammonium persulfate; peroxides such as hydrogen peroxide, t-butyl peroxide, and methylethyl-ketone peroxide; azo compounds such as 2,2'-azobis (2-amidinopropane) dihydrochloride, and 2,2'-azobis [2-(2-imidazoline 2-yl) propane] dihydrochloride.

Examples of the redox polymerization initiators encompass systems each of which is a combination of (i) a reducing compound such as L-ascorbic acid or sodium hydrogen sulfite and (ii) the foregoing persulfate or peroxide.

Further, it is also a preferable embodiment to use the photodegradable polymerization initiator and the pyrolytic polymerization initiator in combination. Still further, an active energy ray such as an ultraviolet ray, an electron ray, or a gamma ray may be used alone or used in combination with the polymerization initiator.

The amount of the polymerization initiator to be used is preferably in a range from 0.0001 mol% to 1 mol%, and more preferably 0.0005 mol% to 0.5 mol%, relative to the total amount of the monomers. The polymerization initiator used in an amount of more than 1 mol% may cause deterioration in color tone of water absorbent resin powder. Further, the polymerization initiator used in an amount of less than 0.0001 mol% may increase residual monomers.

(More preferable polymerization method)

As the polymerization method of an acrylic acid (salt)-based monomer aqueous solution according to the present invention, at least one of reverse-phase suspension polymerization, spraying polymerization, droplet polymerization, and aqueous polymerization, and particularly aqueous polymerization, are employed from the viewpoint of physical properties (for example, water absorbing speed and liquid permeability) of water absorbent resin powder and polymerization controllability, and others.

Examples of a preferable aspect of the aqueous polymerization encompass high-temperature starting aqueous polymerization, high-concentration aqueous polymerization, and high-concentration high-temperature starting aqueous polymerization. The high-temperature starting aqueous polymerization is such that a polymerization starting temperature is preferably not lower than 40 °C, more preferably not lower than 50 °C, still more preferably not lower than 65 °C, particularly preferably not lower than 70 °C, and most preferably not lower than 80 °C (upper limit is a boiling point). The high-concentration aqueous polymerization is such that a monomer concentration is preferably not less than 40 mass%, more preferably not less than 45 mass%, and still more preferably not less than 50 mass% (upper limit is not more than 90 mass%, preferably not more than 80 mass%, and more preferably not more than 70 mass%). The high-concentration high-temperature starting aqueous polymerization is the combination of the high-temperature starting aqueous polymerization and the high-concentration aqueous polymerization.

As a polymerization scheme to be employed, kneader polymerization or belt polymerization is preferable. Examples of a preferable scheme of aqueous polymerization encompass continuous belt polymerizations (disclosed in U.S. Patent No. 4893999, U.S. Patent No. 6241928, U.S. Patent Application Publication No. 2005/215734, a pamphlet of International Publication No. WO 2008/114847, etc.), continuous kneader polymerization, batch kneader polymerizations (U.S. Patent No. 6987151, U.S. Patent No. 6710141, a pamphlet of International Publication No. WO 2008/114848).

Further, high-temperature starting continuous aqueous polymerization, high-concentration continuous aqueous polymerization, and high-concentration high-temperature starting continuous aqueous polymerization, all of which are combinations of the preferable aspects and the preferable polymerization schemes, can be exemplified.

As another preferable example, batch polymerization or continuous kneader polymerization such that the polymerization starting temperature is not lower than 15 °C and that the monomer concentration is not lower than 30 mass% can be exemplified.

Moreover, in carrying out the polymerization, a polymerization starting time (a time period between the addition of the polymerization initiator and the initiation of polymerization) is preferably more than 0 second but not more than 300 seconds, and more preferably in a range from 1 second to 240 seconds.

By employing the aforementioned aqueous polymerization, it is possible to produce water absorbent resin powder with high productivity. It should be noted that the above polymerization methods are preferably employed in a huge-scale manufacturing device whose production amount per line is large. The production amount is preferably not less than 0.5 [t/hr], more preferably not less than 1 [t/hr], still more preferably not less than 5 [t/hr], and particularly preferably not less than 10 [t/hr].

(2-3) Gel-crushing step

The present step is an optional step of carrying out gel-crushing of a water-containing gel-like crosslinked polymer (hereinafter referred to as "hydrogel") obtained through the polymerization step, etc. (particularly, aqueous polymerization), thereby obtaining a particulate hydrogel (hereinafter referred to as "particulate hydrogel").

The hydrogel obtained through the aqueous polymerization is gel-crushed, especially by mixing and kneading for grain refining, in order to attain both of water absorbing speed and liquid permeability of a resultant water absorbent resin and to further improve impact resistance of the resultant water absorbent resin. That is, in order to attain the object of the present invention, it is preferable to adopt the aqueous polymerization rather than the reverse-phase suspension polymerization in which the gel-crushing is not necessary. It is particularly preferable to adopt the aqueous polymerization such that gel-crushing is carried out during polymerization (for example, kneader polymerization) or after polymerization (for example, belt polymerization, and, if necessary kneader polymerization).

A gel-crushing device applicable to the present invention is not particularly limited but is, for example, batch-type or continuous gel crusher having a plurality of rotational stirring blades such as double-armed kneader, a single- or twin-screwed extruders, meat chopper, etc. can be adopted. Among them, a screwed extruder having a porous plate at an end is preferable. Examples of the screwed extruder having a porous plate at an end encompass a screwed extruder disclosed in Japanese Patent Application Publication, Tokukai, No. 2000-63527.

In the gel-crushing step of the present invention, a temperature of the hydrogel (gel temperature) before gel-crushing is preferably in a range from 60 °C to 120 °C, and more preferably 65 °C to 110 °C, from the viewpoint of particle size control of a particulate hydrogel and physical properties of a water absorbent resin. A gel temperature lower than 60 °C results in a greater hardness of the resultant hydrogel due to the property of a hydrogel, thereby making it difficult to control the particle shape and particle size distribution in gel-crushing. Moreover, a gel temperature higher than 120 °C results in a greater softness of the resultant hydrogel, thereby making it difficult to control the particle shape and particle size distribution. It should be noted that the gel temperature can be controlled by a temperature during the polymerization, heating after the polymerization, cooling after the polymerization, or others.

Further, mass average particle diameter (D50) (defined by sieve classification) of a particulate hydrogel after the gel-crushing is preferably in a range from 0.5 mm to 3 mm, more preferably in a range from 0.6 mm to 2 mm, and still more preferably in a range from 0.8 mm to 1.5 mm. Moreover, the proportion of a coarse particulate hydrogel of not less than 5 mm in particle diameter is preferably not more than 10 mass%, more preferably not more than 5 mass%, and still more preferably not more than 1 mass%, relative to a total amount of the pariculate hydrogel.

In the present invention, the polymerization step and gel-crushing step can be carried out by any of the following methods: kneader polymerization method of carrying out gel-crushing of a water-containing gel-like crosslinked polymer obtained during the polymerization; and a method of subjecting a water-containing gel-like crosslinked polymer obtained by the continuous belt polymerization to the gel-crushing step.

(2-4) Drying step

The present step is a step of drying a hydrogel obtained through the polymerization step, etc. to obtain dried polymer. In a case where the aqueous polymerization is carried out in the polymerization step, a hydrogel is gel-crushed (grain-refined) before and/or after dried. Further, the dried polymer (aggregates) obtained by the drying step may be supplied directly to the pulverization step.

A drying method employed in the present step is not particularly limited and can therefore be a variety of methods. Specifically, as the drying method, drying by heating, hot-air drying, drying under reduced pressure, infrared drying, microwave drying, azeotropic dehydration with a hydrophobic organic solvent, high humidity drying with use of high-temperature vapor, and the like method can be used solely or two or more of these methods can be used in combination. A drying temperature is preferably in a range from 100 °C to 300 °C, and more preferably in a range from 150 °C to 250 °C.

Further, a drying time is not particularly limited because it depends on the surface area of and the moisture content in the hydrogel, the type of drying apparatus, and the like. For example, the drying time is preferably in a range from 1 minute to 5 hours, and more preferably in a range from 5 minutes to 1 hour. Further, a resin solid content is preferably not less than 80 mass%, more preferably in a range from 85 mass% to 99 mass%, and still more preferably in a range from 90 mass% to 98 mass%, where the resin solid content is calculated from weight loss caused by drying (change in mass of 1 g of powder or particles before and after drying at 180 °C for 3 hours).

(2-5) Pulverization step, classification step

The present step is a step of pulverizing and/or classifying a dried polymer obtained in the drying step and is preferably a step of obtaining a water absorbent resin powder having a specific particle size. It should be noted that the present step is different from the above (2-3) gel-crushing step in that a product to be pulverized has undergone the drying step. Further, a water absorbent resin obtained after the pulverization step may be referred to as a "pulverized resin".

(Particle size distribution)

The mass average particle diameter (D50) of a water absorbent resin powder before surface-crosslinked is preferably in a range from 200 $\mu$m to 600 $\mu$m, more preferably in a range from 200 $\mu$m to 550 $\mu$m, still more preferably in a range from 250 $\mu$m to 500 $\mu$m, and particularly particularly in a range from 350 $\mu$m to 450 $\mu$m, from the viewpoint of water absorbing speed, liquid permeability, absorption capacity under load, etc. of a resultant water absorbent resin. Further, the fewer fine particles having a particle diameter of less than 150 $\mu$m defined by standard sieve classification, the better. The fine particle content is preferably in a range from 0 to 5 mass%, more preferably in a range from 0 to 3 mass%, and still more preferably in a range from 0 to 1 mass%, from the viewpoint of liquid permeability, etc. of a resultant water absorbent resin.

Still further, the fewer coarse particles having a particle diameter of not less than 850 $\mu$m defined by standard sieve classification, the better. The coarse particle content is preferably in a range from 0 mass% to 5 mass%, more preferably 0 mass% to 3 mass%, and still more preferably 0 to 1 mass%, from the viewpoint of water absorbing speed, etc. of a resultant water absorbent resin. Yet further, the amount of large particles having a particle diameter of not less than 710 $\mu$m is preferably in a range from 0 to 20 mass%, more preferably in a range from 0 to 10 mass%, still more preferably in a range from 0 to 5 mass%, further still more preferably in a range from 0 to 3 mass%, and particularly preferably in a range from 0 to 1 mass%.

Moreover, as to a particle diameter distribution range, particles having particle diameters of preferably not less than 150 $\mu$m but less than 850 $\mu$m, more preferably not less than 150 $\mu$m but less than 710 $\mu$m, are preferably contained in an amount of not less than 95 mass%, more preferably in an amount of not less than 98 mass%, and still more preferably in an amount of not less than 99 mass% (upper limit is 100 mass%), from the viewpoint of water absorbing speed, liquid permeability, absorption capacity under load, etc. of a resultant water absorbent resin.

Controlling the mass average particle diameter or the particle diameter (hereinafter also referred to simply as "particle size") of a water absorbent resin powder can be performed in the polymerization step, the gel-crushing step, or the pulverization/classification step after the drying step. Especially, such control is preferably performed in the classification step after the drying. Further, the measurement of the particle size is carried out with use of JIS standard sieves (Z8801-1 (2000)) in accordance with the method defined in International Publication No. WO 2004/69915 or EDANA-ERT420.2-02.

The water absorbent resin powder in accordance with the present invention may be in the form of spheres, in the form of aggregate of the spheres, or in the form of irregular fragments obtained through the pulverization step subjected to the hydrogel or the dried polymer. However, from the viewpoint of water absorbing speed of a resultant water absorbent resin, it is preferable that the water absorbent resin powder is in the form of irregular fragments or in granulated form of the irregular fragments.

In order to further attain the object of the present invention, the particle size is applied preferably to a particle size after the surface crosslinking, and further preferably to the water absorbing agent, which is the final product.

(2-6) Fine powder recycling step

The production method in accordance with the present invention preferably includes, after the drying step, the classification step (including the second classification step performed after the surface crosslinking step; hereinafter the same applies to the following descriptions) of separating a water absorbent resin fine particle passing through a standard sieve having a mesh size of 150 $\mu$m, after which the water absorbent resin fine particle or a mixture thereof with water is recycled (reused) in the steps which are performed before the drying step. It should be noted that the coarse particles removed in the classification step may be re-pulverized if necessary. Moreover, the fine particles removed in the classification step may be discarded, used for another purposes, or provided for use in the fine powder recycling step.

Removing the fine particles makes it possible to improve liquid permeability (for example, SFC) of a resultant water absorbent resin. Further, the present step makes it possible to further improve water absorbing speed (for example, FSR).

That is, in the production method in accordance with the present invention, the fine powder recycling step refers to a step of recycling water absorbent resin fine particles (containing, in particular, particles having a particle diameter of not more than 150 $\mu$m in amount of not less than 70 mass%; hereinafter also referred to as "fine powder"), which are generated in the drying step, and if necessary, in the pulverization step and in the classification step and then separated out, in such a manner that the fine powder is directly recycled or recycled in the hydrated or granulated form in any of the steps before the drying step, preferably in the polymerization step, the gel-crushing step, or the drying step.

By recycling the fine powder, it is possible to control particle sizes of a water absorbent resin and a water absorbing agent and to further improve the water absorbing speed of a water absorbent resin obtained by the present step.

The fine powder to be recycled may be a fine powder before or after the surface crosslinking. The amount of the recycled fine powder is preferably in a range from 1 mass% to 40 mass%, and more preferably in a range from 5 mass% to 30 mass% relative to the total mass of dried polymer.

A preferable fine powder recycling method in the present invention is a method in which the water absorbent resin fine particle or a product in hydrated or granulated form of the water absorbent resin fine particle, and if necessary, inorganic fine particles and others are mixed into the monomer aqueous solution to be polymerized, the hydrogel during the polymerization, or a drying machine used in the drying step. A method of recycling the fine powder into the monomer aqueous solution to be polymerized is exemplified by the methods disclosed in PCT international publications Nos. 92/001008 and 92/020723. A method of recycling the fine powder into the hydrogel during the polymerization is exemplified by the methods disclosed in PCT international publications Nos. 2007/074167, 2009/109563, 2009/153196, and 2010/006937. Further, a method of performing recycling in the drying step (into a drying machine) is exemplified by the method disclosed in U.S. Patent No. 6228930, etc. These fine powder recycling methods can be adopted suitably as the fine powder recycling method in the present invention.

(2-7) Surface crosslinking agent addition step

The present step is a step of preparing a water absorbent resin powder containing a surface crosslinking agent provided in the surface crosslinking step. In common practice, surface crosslinking is carried out, for example, by adding an organic surface crosslinking agent described later, by polymerizing the monomer(s) on a surface of a water absorbent resin powder, by adding a radical polymerization initiator such as persulfate and then heating or ultraviolet irradiation, or the like. In the surface crosslinking agent addition step of the present invention, it is preferable that the organic surface crosslinking agent is added to the water absorbent resin powder obtained in the above classification step and further to a water absorbent resin powder obtained in the fine powder recycling step. Further, surface crosslinking may be carried out simultaneously with a liquid permeability enhancer addition step described later.

(Organic surface crosslinking agent)

Examples of the organic surface crosslinking agent usable in the present invention encompass polyhydric alcohol compounds, epoxy compounds, polyhydric amine compounds, condensates of a polyhydric amine compound and a haloepoxy compound, oxazoline compounds, (mono)oxazolidinone compounds, (di)oxazolidinone compounds, (poly)oxazolidinone compounds, oxetane compounds, alkylene carbonate compounds, and the like, from the viewpoint of physical properties of a resultant water absorbent resin powder. Among them, it is particularly preferable to employ a dehydration reaction crosslinking agent including any of a polyhydric alcohol compound, an alkylene carbonate compound, an oxazolidinone compound, which require high-temperature dehydration reaction.

The dehydration reaction surface crosslinking agent is a surface crosslinking agent causing dehydrative esterification reaction of a carboxyl group, which is a functional group of a polyacrylic acid (salt)-based water absorbent resin powder, with a hydroxyl group, which is a functional group of the surface crosslinking agent, or causing dehydrative amidation reaction of the carboxyl group with an amino group, which is a functional group of the surface crosslinking agent. Cyclic surface crosslinking agents, like the alkylene carbonate compounds and the oxazolidinone compounds, with which a hydroxyl group and an amino group are generated in the process of the reaction are also classified as the dehydration reaction surface crosslinking agent.

Specific examples of the organic surface crosslinking agent encompass: polyalcohol compounds such as (di)ethylene glycol, (tri)ethylene glycol, (tetra)ethylene glycol, (poly)ethylene glycol, (di)propylene glycol, (poly)propylene glycol, 1,3-propanediol, 2,2,4-trimethyl-1,3-pentanediol, (poly)glycerin, 2-butene-1,4-diol, 1,4-butanediol, 1,3-butanediol, 1,5-pentanediol, 1,6-hexanediol, trimethylolpropane, diethanolamine, triethanolamine, pentaerythritol, and sorbitol; epoxy compounds such as (poly)ethylene glycol diglycidyl ether, (di)glycerol polyglycidyl ether, (poly)glycerol polyglycidyl ether, and glycidol; oxazoline compounds such as 2-oxazolidone, N-hydroxyethyl-2-oxazolidone, and 1,2-ethylene bisoxazoline; alkylene carbonate compounds such as 1,3-dioxolane-2-one, 4-methyl-1,3-dioxolane-2-one, 4,5-dimethyl-1,3-dioxolane-2-one, 4,4-dimethyl-1,3-dioxolane-2-one, 4-ethyl-1,3-dioxolane-2-one, 4-hydroxymethyl-1,3-dioxolane-2-one, 1,3-dioxane-2-one, 4-methyl-1,3-dioxane-2-one, 4,6-dimethyl-1,3-dioxane-2-one, and 1,3-dioxopane-2-one; haloepoxy compounds such as epichlorohydrin, epibromohydrin and α-methylepichlorohydrin and polyhydric amine adducts thereof (for example, Kymene® manufactured by Hercules); silane coupling agents such as γ-glycidoxypropyltrimethoxysilane and γ-aminopropyltriethoxysilane; oxetane compounds such as 3-methyl-3-oxetane methanol, 3-ethyl-3-oxetane methanol, 3-butyl-3-oxetane methanol, 3-methyl-3-oxetane ethanol, 3-ethyl-3-oxetane ethanol, 3-butyl-3-oxetane ethanol, 3-chloromethyl-3-methyloxetane, 3-chloromethyl-3-ethyloxetane, and polyhydric oxetane compounds; cyclic urea compounds such as 2-imidazolidinone.

Among these, the organic surface crosslinking agent is preferably selected from the polyhydric alcohol compounds, the epoxy compounds, the oxazoline compounds, and the alkylene carbonate compounds. More preferably, the organic surface crosslinking agent is a combination of any compound selected from the polyhydric alcohol compounds and any compound selected from organic surface crosslinking agents (epoxy compounds, oxazolinone compounds, and the alkylene carbonate compounds) other than polyhydric alcohols.

From the viewpoint of higher physical properties of a resultant water absorbent resin powder, it is preferable that the organic surface crosslinking agent is a combination of particularly a polyhydric alcohol and a compound (preferably, an epoxy compound or an alkylene carbonate, and particularly preferably, an alkylene carbonate) other than the polyhydric alcohols. As a suitable surface crosslinking method, any of the methods described in Patent Literature 45 (pamphlet of International Publication No. WO 2012/102406) and Patent Literature 46 (pamphlet of International Publication No. WO 2012/102407) is employed.

The organic surface crosslinking agent is preferably a combination of a plurality compounds selected from the polyhydric alcohols, the alkylene carbonates, the oxazolidinone compounds, the oxetane compounds, and amino alcohol compounds, more preferably a combination of particularly the polyhydric alcohols and cyclic compounds selected from the alkylene carbonates, the oxazolidinone compounds, and the oxetane compounds, and still more preferably a combination of any polyhydric alcohol and any alkylene carbonate as described in Patent Literatures 45 and 46. Particularly preferably, any of the methods described in (2-8), (2-9), and (3-3) to (3-9) of Patent Literatures 45 and 46 is employed as the surface crosslinking agent addition step of the present invention, and these descriptions shall be descriptions of the present application.

The polyhydric alcohols are C2-C8 polyhydric alcohols, preferably C3-C6 polyhydric alcohols, and particularly preferably C3 or C4 polyhydric alcohols. Specifically, the polyhydric alcohols are preferably, for example, diols, and examples of the diols encompass ethylene glycol, propylene glycol, and 1,3-propanediol, 1,4-butanediol.

In a case where the dehydration reaction surface crosslinking agent is a combination of any of the polyhydric alcohols and any surface crosslinking agent other than the polyhydric alcohols, a ratio (mass ratio) of any of the polyhydric alcohols to any surface crosslinking agent other than the polyhydric alcohols is typically 1:9 to 9:1, preferably 2:8 to 8:2, more preferably 3:7 to 7:3, and particularly preferably 5:5 to 7:3, wherein the ratio is a ratio of the mass of the polyhydric alcohol to the total mass of the material other than the polyhydric alcohol in the dehydration reaction surface crosslinking agent. The material other than the polyhydric alcohol in the dehydration reaction surface crosslinking agent is preferably the cyclic compound, more preferably an alkylene carbonate, and still more preferably an ethylene carbonate.

The polyhydric alcohol compound as used in the present invention is suitably propylene glycol, 1,3-propanediol, and 1,4-butanediol. Further, the epoxy compound as used in the present invention is suitably a polyglycidyl compound. The oxazoline compound as used in the present invention is suitably 2-oxazolidinone, and the alkylene carbonate compound as used in the present invention is suitably 1,3-dioxolane-2-one.

A temperature of a solvent to be mixed into the organic surface crosslinking agent is appropriately determined. A solvent being too low in temperature may result in too low solubility and too high viscosity. Especially, in a case where a solid non-polymerized organic compound described later is used as the surface crosslinking agent, particularly, ethylene carbonate is used as the surface crosslinking agent, it is preferable that the solvent as used is water warmed to room temperature or higher (preferably in a range from 30 °C to 100 °C, more preferably in a range from 35 °C to 70 °C, and still more preferably 40 °C to 65 °C).

That is, it is preferable that other compound, water in particular, to be mixed with a non-polymerized organic compound (particularly, a solid surface crosslinking agent, and further, a solid polyhydric alcohol and a solid cyclic compound such as alkylene carbonate) is warmed, and it is more preferable that the other compound is in the aforementioned temperature range.

The alkylene carbonate compound or the polyhydric alcohol compound, particularly a solid alkylene carbonate compound, is preferably heated in advance before mixture with water. A temperature of the compound thus heated is preferably a temperature higher than the temperature of a surface crosslinking agent solution having water added thereto. Specifically, in a case where the solid alkylene carbonate compound is used, it is preferable that the polyhydric alcohol, particularly the solid polyhydric alcohol is also heated and melted to a temperature preferably in a range from 30 °C to 100 °C, more preferably in a range from 35 °C to 70 °C, and still more preferably 40 °C to 65 °C. (Mixture ratio control)

A mixture control ratio of the surface crosslinking agent solution in the present invention depends on subtle variation in concentration and ratio of the surface crosslinking agent solution. Particularly, the subtle variation in concentration and ratio of the surface crosslinking agent solution may occur due to air temperature changes every day or every season. For this reason, it is preferable that the mixture of the surface crosslinking agent solution in the present invention into a water absorbent resin powder is performed while a flow rate is measured with a mass flowmeter, particularly Coriolis-type mass flowmeter.

When a moving mass encounters vibration vertical to a moving direction, a Coriolis force in response to a velocity of the mass is generated. The Coriolis mass flowmeter is provided with a resonating, measuring tube for correctly generating this effect, and when a fluid (=mass) moves in the measuring tube, a Coriolis force is generated. By sensors at an outlet and an inlet, slippage of a vibration phase of a measuring tube is sensed, and a microprocessor analyzes and uses this information to calculate a mass flow rate. Further, by a resonating frequency of the measuring tube, direct measurement of a fluid density is possible, and a temperature of the measuring tube is also measured for correcting influence of a temperature. This signal corresponds to a temperature of a process, and can be also used as an output signal. The Coriolis-type mass flowmeter is suitably used not only in preparation of a surface crosslinking agent at a predetermined ratio, but also in mixture of a surface crosslinking agent into a water absorbent resin after the preparation.

(Solvent and concentration)

In a case where the organic surface crosslinking agent is used, the organic surface crosslinking agent is used preferably in an amount (total amount throughout the addition step) of 0.001 to 15 parts by mass, and more preferably 0.01 to 5 parts by mass, relative to 100 parts by mass of the water absorbent resin before the addition of the organic surface crosslinking agent.

In a case where the organic surface crosslinking agent as used is two types of compounds, i.e. a polyhydric alcohol compound and a compound selected from among compounds other than polyhydric alcohol compounds, the polyhydric alcohol compound is used preferably in an amount (total amount throughout the addition step) of 0.001 to 10 parts by mass, and more preferably 0.01 to 5 parts by mass, relative to 100 parts by mass of the water absorbent resin before the addition of the organic surface crosslinking agent. Further, the compound other than the polyhydric alcohol compound is used in an amount (total amount throughout the addition step) of 0.001 to 10 parts by mass, and more preferably 0.01 to 5 parts by mass relative to 100 parts by mass of the water absorbent resin before the addition of the organic surface crosslinking agent.

The surface crosslinking agent solution preferably contains water. That is, it is preferable that the surface crosslinking agent solution is an aqueous surface crosslinking agent solution. The water is used preferably in an amount (total amount throughout the addition step) of 0.5 to 20 parts by mass, and more preferably 0.5 to 10 parts by mass, relative to 100 parts by mass of the water absorbent resin before the addition of the organic surface crosslinking agent. It should be noted that the water also includes crystalline water, hydrated water, or the like of the surface crosslinking agent.

Further, a hydrophilic organic solvent may be used in the surface crosslinking agent addition step. The hydrophilic organic solvent is used preferably in an amount of more than 0 part by mass but not more than 10 parts by mass, more preferably in an amount of more than 0 part by mass but not more than 5 parts by mass, relative to 100 parts by mass of the water absorbent resin before the addition step. Examples of the hydrophilic organic solvent encompass a $C_1$-$C_4$ primary alcohol, a $C_2$-$C_3$ primary alcohol, lower ketones whose carbon number is 4 or lower, such as acetone, and the like. In particular, volatile alcohols each having a boiling point of lower than 150 °C, more preferably lower than 100 °C, are more preferable as the hydrophilic organic solvent, because the volatile alcohols evaporate during the surface crosslinking and therefore no residue will remain.

Specifically, examples of the hydrophilic organic solvent encompass: lower alcohols such as methyl alcohol, ethyl alcohol, n-propyl alcohol, isopropyl alcohol, n-butyl alcohol, isobutyl alcohol, and t-butyl alcohol; ketones such as acetone; ethers such as dioxane, tetrahydrofuran, and methoxy (poly)ethylene glycol; amides such as epsilon-caprolactam and N,N-dimethylformamide; sulfoxides such as dimethyl sulfoxide; polyhydric alcohols such as polyoxypropylene and oxyethylene-oxypropylene block copolymers.

Further, in mixing a surface crosslinking agent solution into a water absorbent resin powder, water-insoluble fine particles and a surfactant may be added in an amount within a range such that the effect of the present invention is not interfered with. Specifically, the water-insoluble fine particles and the surfactant can coexist in an amount of more than 0 part by mass but not more than 10 parts by mass, preferably more than 0 part by mass but not more than 5 parts by mass, and more preferably more than 0 part by mass but not more than 1 part by mass, relative to 100 parts by mass of a water absorbent resin before the addition step. In this case, a surfactant or the like as used in the present invention can be the one disclosed in U.S. Patent No. 7473739.

A concentration of a surface crosslinking agent in the surface crosslinking agent solution is appropriately determined. The surface crosslinking agent solutions in the present invention can be aqueous surface crosslinking agent solutions such that the surface crosslinking agent concentration based on a total amount of all of the surface crosslinking agents in the individual surface crosslinking agent solutions used in all of the addition processes, is in a range from 1 mass% to 80 mass% and is further in a range from 5 mass% to 60 mass%, in a range from 10 mass% to 40 mass%, or in a range from 15 mass% to 30 mass%. It should be noted that the hydrophilic organic solvent and/or other component(s) may be contained as a residue.

A temperature of the surface crosslinking agent solution is appropriately determined based on solubility of a surface crosslinking agent to be used, viscosity of the surface crosslinking agent solution, or the like. The temperature of the surface crosslinking agent solution is preferably in a range from -10 °C to 100 °C, more preferably in a range from 5 °C to 70 °C, still more preferably in a range from 10 °C to 65 °C, and particularly preferably in a range from 25 °C to 50 °C. The surface crosslinking agent solution having a high temperature is not preferable because it can cause, before mixed or reacted with a water absorbent resin powder, the followings: (1) In a case where the surface crosslinking agent is a cyclic surface crosslinking agent, the cyclic surface crosslinking agent is hydrolyzed (e.g. degradation from ethylene carbonate into ethylene glycol, degradation from oxazolidinone into ethanolamine); and (2) mixability is deteriorated by volatilization and the like of water and a hydrophilic organic solvent contained in the surface crosslinking agent solution. The surface crosslinking agent solution having a low temperature may cause (1) coagulation of the surface crosslinking agent solution and (2) precipitation of the surface crosslinking agent. (Surfactant)

A polyacrylic acid (salt)-based water absorbent resin powder of the present invention may contain a surfactant, and it is preferable that a surfactant is mixed in any of the steps included in the production method according to the present invention.

By coating the surface of the water absorbent resin powder in the present invention with a surfactant, it is possible to obtain a water absorbent resin powder having a high water absorbing speed and a high liquid permeability. It should be noted that the surfactant used in the present invention is not particularly limited, but examples of the surfactant encompass surfactants disclosed in International Publication No. WO 97/017397 and U.S. Patent No. 6107358, i.e. nonionic surfactants, anionic surfactants, cationic surfactants, amphoteric surfactants. These surfactants may have polymerizability or reactivity with an acrylic acid (salt)-based monomer or a water absorbent resin powder. As a specific compound for the surfactant, compounds described in (2-1) of Patent Literatures 45 and 46 are employed.

The type and use amount of a surfactant to be used is determined appropriately. The surfactant in the present invention is used preferably in a range such that the surface tension described in U.S. Patent No. 2006/204755 is obtained. Specifically, the use amount of the surfactant in the present invention is in a range from 0 to 0.5 parts by weight, 0.00001 to 0.1 parts by weight, or 0.001 to 0.05 parts by weight, relative to a water absorbent resin. Among the aforementioned surfactants, anionic surfactants, nonionic surfactants, or silicone surfactants are preferably used. More preferably, nonionic surfactants or silicone surfactants are used.

(Use of a surface crosslinking agent solution in combination with an acid or a base)

For the purpose of promoting reaction and uniform mixture of a surface crosslinking agent, the surface crosslinking agent solution may contain not only the organic surface crosslinking agent, the hydrophilic organic solvent, the surfactant, and water-insoluble fine particles, but also an acid or a base.

As the acid or the base, an organic acid or a salt thereof, an inorganic acid or a salt thereof, and an inorganic base can be used. The acid or the base is appropriately used in an amount in a range from 0 to 10 parts by mass, more preferably in a range from 0.001 to 5 parts by mass, and still more preferably 0.01 to 3 parts by mass, relative to 100 parts by mass of the water absorbent resin before added with the surface crosslinking agent solution. The organic acid is a $C_1$-$C_6$, more preferably a $C_2$-$C_4$, water-soluble organic acid, water-soluble saturated organic acid, and saturated organic acid containing a hydroxyl group, and particularly preferably a saturated organic acid containing a hydroxyl group.

Another examples of the acid or the base encompass; non-crosslinkable, water-soluble inorganic bases (preferably, an alkali metal salt, an ammonium salt, a hydroxide of an alkali metal, and an ammonia or a hydroxide thereof); an irreducible-alkali-metal-salt pH buffer (preferably bicarbonate, dihydrogen phosphate, hydrogen phosphate etc.); and the like.

(Method by which to add a surface crosslinking agent solution)

By an addition process, the surface crosslinking agent is added to a water absorbent resin powder. Examples of a method for the addition process, which is not particularly limited, include: (1) a method of immersing a water absorbent resin into a hydrophilic organic solvent so as to cause a surface crosslinking agent to be adsorbed to the water absorbent resin; (2) a method of spraying or dropping a surface crosslinking agent solution directly to a water absorbent resin so as to mix the water absorbent resin with the surface crosslinking agent solution; and the like. From the viewpoint of evenly adding a predetermined amount of surface crosslinking agent to a water absorbent resin, the method (2) is preferable. Further, it is preferable that the addition process is performed while the water absorbent resin is stirred in order that the surface crosslinking agent is evenly added to the water absorbent resin. Spraying the surface crosslinking agent is more preferable.

In a case where two or more types of surface crosslinking agents having mutually different compositions are used in the addition process, different spray nozzles, for example, may be used for simultaneous addition of the surface crosslinking agents. However, from the viewpoint of evenly adding the surface crosslinking agents, it is preferable that before the addition, these surface crosslinking agents are adjusted into a surface crosslinking agent having a single composition. Further, in a case where the surface crosslinking agent has a single composition, a plurality of spray nozzles may be used in consideration of size and throughput of an apparatus to be used in the addition process, spraying angle of a spray nozzle, and others.

Preferable examples of an apparatus for use in the addition process (hereinafter also referred to as "mixing apparatus) encompass, cylindrical mixers, double-wall conical mixers, V-shaped mixers, ribbon mixers, screw type mixers, fluidization type furnaces, rotary disc mixers, air mixtures, double-arm kneaders, internal mixers, pulverizing type kneaders, rotating mixers, screw-type extruders, Turbulizer, ploughshare mixers, and the like. Further, in large-scale production such as commercial production, the mixing apparatus is preferably an apparatus capable of performing continuous mixture. Still further, one and the same apparatus may be used in each of the addition processes, or separate apparatuses may be used in the addition processes.

The water absorbent resin powder used in the present step is preferably heated and warmed. The temperature of the water absorbent resin powder is preferably in a range from 30 °C to 100 °C, more preferably in a range from 35 °C to 80 °C, and still more preferably in a range from 40 °C to 70 °C. A water absorbent resin powder having a low temperature causes precipitation of a surface crosslinking agent, moisture adsorption of a water absorbent resin, or the like, which may result in inadequate or uneven surface treatment or the like to the water absorbent resin powder. A water absorbent resin powder having an extremely high temperature, particularly a temperature higher than a boiling point of water in a case where a surface crosslinking agent solution is an aqueous surface crosslinking agent solution, causes evaporation or the like of water contained in the aqueous surface crosslinking agent solution, which may in turn precipitation or the like of the surface crosslinking agent. The temperature of a mixture obtained through the present step, i.e. a mixture of the surface crosslinking agent solution and a water absorbent resin powder, is preferably in a range from 30 °C to 100 °C, more preferably in a range from 30 °C to 90 °C, and still more preferably in a range from 30 °C to 80 °C. The mixture having a temperature falling within the above range yields the following effect: an added surface crosslinking agent can be effectively reacted in the surface crosslinking step described later and also keep an appropriate fluidity.

(2-8) Surface crosslinking step

The present step is a step of performing heat treatment for crosslinking the surface of the water absorbent resin powder or the periphery of the surface of the water absorbent resin powder, in order to improve absorption capacity under load and liquid permeability of a water absorbent resin powder. The present step can be carried out simultaneously with the surface crosslinking agent addition step or after the surface crosslinking agent addition step. The present step is preferably carried out after the surface crosslinking agent addition step, from the viewpoint of quality stabilization. In a production method according to the present invention, the present step may be carried out once or may be carried out twice or more times under one and the same condition or under different conditions. It should be, however, noted that it is possible to obtain a water absorbing agent according to the present invention by performing the present step at least once under an atmosphere controlled at a specific dew point.

(Heating apparatus)

A heating apparatus as used in the present invention is exemplified by a continuous type or batch type heating apparatus including (a) a publicly known drier or a publicly known heating furnace and (b) a gas discharge mechanism and/or a gas supply mechanism for causing the drier or the heating furnace to have a predetermined atmosphere. The continuous type heating apparatus is preferable.

A heating method of the heating apparatus is suitably a method of a conductive heat transfer type, a method of a radiative heat transfer type, a method of a hot-air heat transfer type, a method of a dielectric heating type. The heating method is preferably the heating method of a conductive heat transfer type and/or of a hot-air heat transfer type, and more preferably the heating method of a conductive heat transfer type.

A control temperature of the heating apparatus needs only to be a temperature at which a water absorbent resin can be heated to a temperature described later, and needs not to be held constant from the beginning to the end of the surface crosslinking step. In order to prevent partial overheating, the temperature of the heating apparatus is preferably in a range from 100 °C to 300 °C, more preferably in a range from 120 °C to 280 °C, still more preferably in a range from 150 °C to 250 °C, and particularly preferably in a range from 170 °C to 230 °C.

Further, in order to enhance the efficiency of heating and perform an even heat treatment, it is preferable to use an apparatus including a mechanism for continuously stirring and/or fluidizing an object to be heated. A stirring and/or a fluidizing method is preferably a channel stirring method, a method of a screw type, a method of a rotary type, a method of a disc type, a method of a kneading type, a method of a fluidized-bed type. It is more preferable that a stirring method using stirring blades (paddles) and a stirring method based on movement of a heat transfer surface itself by means of a rotary retort furnace. It should be noted that the stirring and/or fluidizing mechanism used for the purpose of performing an even heat treatment need not to be used in a case where the amount of product to be dried is small, for example, in a case where an object to be dried has a thickness of less than 1 cm.

The heating apparatus includes the gas discharge mechanism for discharging vapors generated from an object to be heated and also is able to control atmospheric dew point and temperature of a heating section (the inside of the heating apparatus) by adjusting the gas discharge mechanism, for example, by adjusting the amount of discharge. It should be noted that the heating section is not the so-called heat source such as a heater or an induction coil but a place to increase the temperature of the object to be heated.

The discharge mechanism corresponds to an air exit. If gas is discharged from an outlet of a heated product, the outlet also corresponds to the discharge mechanism. Further, it is preferable the discharge mechanism adjusts the amount of gas discharged therefrom and a pressure of the gas discharged therefrom by means of a blower or the like. Still further, an air exit in the heating apparatus is not limited to one air exit. A plurality of air exit points can be provided in consideration of the size of the heating apparatus and an adjustment status of the dew point and temperature.

The heating apparatus includes the gas supply mechanism and also is able to control an atmospheric dew point and an atmospheric temperature in the heating section by adjusting the gas supply mechanism, for example, by adjusting the amount of supply.

The adjustment in atmospheric temperature can be carried out by using a part of a heating area of the heating section of the heating apparatus, in which part a water absorbent resin as an object to be heated is not provided. In this case, it is possible to increase the efficiency of heating of gas (gas to be supplied) by using a filler such as a metallic ring-shaped object, a metallic mesh-shaped object, a ceramic ring-shaped object, or a ceramic mesh-shaped object.

In terms of stability of the atmospheric temperature and dew point described below, it is preferable that a direction of airflow in the heating section is controlled to be a constant direction. Particularly, in a case where an apparatus capable of continuous processing is used, the airflow is preferably plumb or horizontal with respect to a flow from an input port of an object to be heated toward an output port, more preferably horizontal, still more preferably a counter-current flow and/or a concurrent flow, and particularly preferably a concurrent flow. It should be noted that the term "a given direction" means that a direction of a flow of a substance does not change from a macroscopic viewpoint rather than one and the same direction in every point. For example, a partial and/or temporary turbulent or vortex state of airflow by stirring, etc. is not subject to the control of airflow in the present invention. On the other hand, a state in which gas is drawn from the input port and gas is discharged from the output port is turned in the middle of the heat treatment into a state in which gas is drawn from the output port and is discharged from the input port, such a change can be expressed as being "not a constant direction."

In a case where a large-size heating apparatus includes a plurality of gas discharge ports and a plurality of gas supply ports, particularly in a case where a continuous processing type heating apparatus is used, for example, a point of concurrent flow and a point of counter-current flow may coexist in the heating section, provided that the direction of the airflow does not change over time at the individual points.

The flow rate of the airflow varies depending on a size of an apparatus particularly in continuous production. The flow rate can control the atmospheric temperature and dew point inside the apparatus to fall within a predetermined range. The flow rate is at least more than 0 $Nm^3$/hr but not more than 10000 $Nm^3$/hr, preferably at least more than 0

$Nm^3$/hr but not more than 5000 $Nm^3$/hr, and more preferably at least more than 0 $Nm^3$/hr but not more than 3000 $Nm^3$/hr. Further, a ratio of gas relative to the amount of a water absorbent resin powder to be processed is preferably not more than 3000 $Nm^3$/ton, and more preferably not more than 1000 $Nm^3$/ton. It should be noted that "$Nm^3$" indicates a volume of gas obtained by conversion on a standard-state basis (0 °C, 1 atmosphere) rather than a volume of gas that exists under a condition of 0 °C and 1 atmosphere.

The flow rate and the ratio are values defined by a total flow rate of gas discharged and the weight of a water absorbent resin, before heat-treated, introduced into the apparatus. It should be noted that the ratio may fall outside in a case where continuous production of the apparatus is not in a steady state, such as at the start of an operation of the apparatus and at the end of the operation of the apparatus.

The gas to be supplied may be appropriately reduced or pressurized and may be appropriately heated or cooled. Usually, the gas to be supplied needs only to be supplied under a condition where an air having a temperature around room temperature (e.g. 0 °C to 50 °C) is under substantially atmospheric pressure (101.3 kPa (1 atmosphere) $\pm$10 %, preferably $\pm$5 %, more preferably $\pm$1 %).

It is preferable that a pressure of gas in the heating section is slightly lower than a normal atmospheric pressure (101.3 kPa (1 atmosphere)). Such a pressure differential is preferably in a range from 0 kPa to -10 kPa, more preferably in a range from 0 kPa to -5 kPa, and still more preferably in a range from 0 kPa to -2 kPa, relative to atmospheric pressure.

In making industrial continuous production, it is possible to use a batch processing-type or continuous processing-type heating apparatus including the above mechanism.

In the case of the batch type heating apparatus, a method of standing an object to be heated in one or more trays or the like in a manner so as to be distributed substantially evenly, a method of filling an object to be heated in a single bath or a plurality of baths and then heating the object to be heated while stirring with stirring blades or the like, a method of filling an object to be heated in a fluidized bed and then heating the object to be heated while stirring with stirring blades or the like, or the like method is used. In the case of the continuous processing type heating apparatus, a method of carrying a belt or a plurality of trays on which an object to be heated is distributed substantially evenly, a method of carrying an object to be heated while stirring with stirring blades, a screw, or the like, a method of carrying an object to be heated by an inclined heating surface is used.

Specifically, a heating apparatus used in the present invention is preferably a conductive heat transfer type heating apparatus including a continuous stirring mechanism using pressurized steam (high pressure steam) as a heat source. Further, in order to efficiently perform continuous production, a heating apparatus used in the present invention preferably has a slope (tilted at an angle of more than 0 degree relative to a horizontal plane) by which an object to be heated can be flown down to the output port by gravity flow. If the tilt angle of the slope pointing downward is too large, it may cause variations in heating time. For this reason, the slope of the heating apparatus has a tilt angle of preferably in a range from more than 0 degree to not more than 20 degrees and more preferably in a range from more than 0 degree to not more than 10 degrees, relative to the horizontal plane.

It should be noted that in a case where the addition process is performed before the heat treatment and after the heat treatment, one and the same apparatus as used in the above addition process may be used in these addition processes. Alternatively, separate apparatuses may be used in these addition processes. Particularly, in a case where a production apparatus of continuous type is used, it is preferable in terms of production efficiency that one and the same apparatus is used both in the addition process performed before heating and the heat treatment while another apparatus is used in the addition process performed after heating.

Further alternatively, a plurality of heating apparatuses may be used, wherein one type of combination of the aforementioned heating schemes, stirring schemes, gas discharge schemes, and gas supply schemes or different types of combinations of these schemes are employed in each of the heating apparatuses.

For the control of an atmospheric dew point and temperature described later, the aforementioned gas discharge amount, temperature of gas to be supplied, flow rate, dew point, etc. are appropriately controlled in consideration of (i) heat transfer from wall surfaces of a heating apparatus or from a water absorbent resin and (ii) rise in dew point due to water vapors generated from a water absorbent resin in a heating apparatus.

(Atmospheric dew point and temperature)

The atmospheric dew point and temperature in the present step means atmospheric dew point and temperature of gas that exists in an upper space above an object to be heated in the heating section of the heating apparatus.

A method of adjusting the dew point is exemplified by a (a) method of using, as the gas to be supplied, steam, dry air, nitrogen, helium, argon, and/or dried air, and a (b) method of using water vapor generated from water contained in a water absorbent resin powder when the a water absorbent resin powder is heated in the present step. A specific method of adjusting the dew point is exemplified by, for example, a method of providing a device for measuring a dew point in the heating apparatus so that the dew point can be adjusted as required with the above gas introduced into the heating apparatus, a method of adjusting the dew point by changing the flow rate and pressure of discharge gas derived from the above gas. In the present invention, a plurality of method may be suitably used in combination if necessary.

In order to prevent condensation in the heating section, the atmospheric temperature is preferably a temperature of not lower than the dew point. A specific atmospheric temperature is in a range from 100 °C to 300 °C, more preferably in a range from 100 °C to 250 °C, and still more preferably in a range from 100 °C to 230 °C. It should be noted that the dew point is lower than 45 °C and that a lower limit of the dew point is preferably not lower than -30 °C, more preferably not lower than -10 °C, and still more preferably not lower than 0 °C.

The atmospheric dew point and temperature each change depending upon a location inside the heating section and with a lapse of a processing time. However, the atmospheric dew point and temperature are particularly preferably controlled to fall within given ranges in the apparatus (it is preferable that the atmospheric dew point and temperature do not fall outside the above respective ranges and that a range of a variation in atmospheric dew point and temperature falls within 20 °C, and the variation range falls more preferably within 10 °C, still more preferably within 5 °C, and particularly preferably within 2 °C).

The temperature and dew point are values measured under the above atmosphere in an upper space plumb to a water absorbent resin powder being heated in the heating section. In a case where dew points measured in a plumb direction vary depending on an apparatus used, the highest dew point in the dew points is the dew point in the present invention. It should be noted that the dew point can fall outside the aforementioned range during the step of performing heat treatment (for example, immediately after the introduction of the water absorbent resin powder into the heating section and/or immediately before the discharge from the heating section in the continuous processing method). In order to sufficiently obtain the effect of the present invention, it is necessary only that an atmosphere in the heating section is such that the dew point reaches the above dew point from a point in time when the temperature of a water absorbent resin powder in the heating section, for the first time, reaches 170 °C, more preferably 150 °C, still more preferably 130 °C, and particularly preferably 100 °C onwards.

It should be noted that if the flow rate of gas falls within the above range, a dew point and temperature at an appropriate measurement point inside the gas discharge mechanism of the heating apparatus may be set as the atmospheric dew point and temperature in the present invention. Specifically, no mixture with other gas occurs midway from the heating section to the measurement point, and no treatment is made by means of a gas cleaning device, a compulsory temperature change processing using a heater, a cooler, is not performed, and a time between discharge of gas from the heating section and reach to the measurement point is within one second.

The heat treatment in the surface crosslinking step needs only to be such that a maximum temperature of a water absorbent resin powder as an object to be heated in the surface crosslinking step is higher than an atmospheric dew point of a gas. The maximum temperature is preferably in a range from 175 °C to 300 °C, more preferably in a range from 175 °C to 250 °C, and particularly preferably in a range from 180 °C to 230 °C. If the maximum temperature is lower than 175 °C, covalent bonds for surface crosslinking may be formed insufficiently. If the maximum temperature is higher than 300 °C, a resultant water absorbent resin may be deteriorated. A time period of the heat treatment is not particularly limited, provided that the above temperature condition is satisfied. However, the time period of the heat treatment is usually 1 to 120 minutes and preferably 5 to 60 minutes.

Further, for the purpose of preventing the occurrence of excessive crosslinking reaction and improving handling property in a subsequent step, a water absorbent resin taken out of the heating apparatus may be cooled, as required, to a temperature of preferably lower than 100 °C, and more preferably in a range from 0 °C to 95 °C or in a range from 40 °C to 90 °C.

(2-9) Step of adding an additive

In the production method according to the present invention, it is essential that an additive selected from additives (liquid permeability enhancers), particularly from water-insoluble fine particulate compounds and polyvalent cationic compounds is added. The step of adding an additive selected from the water-insoluble fine particulate compounds and the polyvalent cationic compounds may be carried out simultaneously with the surface crosslinking agent addition step or may be carried out after the surface crosslinking step.

"Carrying out the step of adding an additive simultaneously with the surface crosslinking agent addition step" is any of the following processes: (a) adding the additive which is being mixed into the surface crosslinking agent or the surface crosslinking agent solution; (b) adding the additive simultaneously with the surface crosslinking agent or the surface crosslinking agent solution without mixing with the surface crosslinking agent or the surface crosslinking agent solution; and (c) adding the additive at a stage previous to the surface crosslinking agent addition step, and is also a combination of at least two of these processes.

In a case where the surface crosslinking agent addition step and the additive addition step are each carried out twice or more times, it is more preferable that a last surface crosslinking agent addition step is followed by a last additive addition step, and it is still more preferable that a first surface crosslinking agent addition step is followed by a first additive addition step. It should be noted that in a case where the additive is added only once, the additive addition step is the first addition step and is also the last addition step.

This is exemplified by, for example, the following modes: a mode of carrying out the additive addition step after the surface crosslinking agent addition step; a mode of simultaneously carrying out the surface crosslinking agent addition step and the additive addition step; a mode of simultaneously carrying out the surface crosslinking agent addition step and the additive addition step and then carrying out another additive addition step; and the like.

The additive addition step needs only to be carried out after the first surface crosslinking agent addition step. It is preferable that the additive addition step is carried out at least once after the surface crosslinking agent addition step is carried out at least once, and it is more preferable that the additive addition step is carried out once after all of the surface crosslinking agent addition steps.

The additive used in the present invention is an additive selected from water-insoluble fine particulate compounds and cationic compounds. The additive is used to exert effects of preferably a liquid permeability enhancer and an Anti-Caking agent, particularly preferably a liquid permeability enhancer. In consideration of a representative function of the additives, the additives described above and additives described below in the present invention may also be collectively referred hereinafter to as "liquid permeability enhancers."

(Liquid Permeability Enhancer)

The liquid permeability enhancer in the present invention is an additive selected from insoluble fine particulate compounds and polyvalent cationic compounds or an additive for increasing SFC or free swelling GBP (preferably obtaining SFC increased to fall within a range described below) as compared with SFC or free swelling GBP obtained when no liquid permeability enhancer is used. It should be noted that the term "GBP" is defined in WO 2004/096304.

The water-insoluble fine particulate compounds and cationic compounds in the present invention serve as a stereoscopic spacer or an electrostatic spacer on the surface of a water absorbent resin. The water-insoluble microparticulate compounds and cationic compounds in the present invention cause a resultant water absorbing agent to have "increased liquid permeability (for example, SFC (described later) increased by not less than $1 \times 10^{-7}$, and further not less than $10 \times 10^{-7}$ as compared with SFC obtained when these compounds are not used)," "increased Anti-Caking property (for example, blocking tendency at moisture absorption (described later) increased by not less than 1 % or by not less than 5 %)," "increased gel strength," "increased free swelling capacity (FSC) (for example, FSC (defined by ERT440.2-02) increased by 0.5g/g or by not less than 1g/g)." Besides, these additives, depending on their kinds, can achieve effects such as "deodorization/antibacterial activity" and "reduction of a residual surface crosslinking agent," but their effects and intended uses are not particularly limited in the present invention.

The additive or liquid permeability enhancer essentially added in the production method according to the present invention is preferably selected from water-insoluble inorganic fine particles and polyvalent cationic compounds (cationic polymer compounds or water-soluble polyvalent metal cation-containing compounds). The "water-soluble" compound as used herein refers to a compound that dissolves, in 100 g of water (25 °C), in an amount of not less than 1 g or not less than 5 g. The "water-insoluble" compound refers to a compound that dissolves, in 100 g of water (25 °C), in an amount of less than 1 g, less than 0.5 g or less than 0.1 g.

(Inorganic fine particles)

Examples of the inorganic fine particles include: water-insoluble fine particulate inorganic powder such as silicon dioxide, titanium dioxide, aluminium oxide, magnesium oxide, zinc oxide, talc, metal phosphate (e.g. calcium phosphate, barium phosphate, and aluminum phosphate), metal borate (e.g. titanium borate, aluminum borate, iron borate, magnesium borate, manganese borate, and calcium borate), silicic acid or salt thereof, clayey materials, diatomaceous earth, zeolite, bentonite, kaolin, hydrotalcite, and activated clay; and organic fine powders such as calcium lactate, aluminum lactate, a metal soap (polyvalent metal salt of long chain fatty acid). As for the inorganic fine particles, volume average particle diameter thereof is preferably not more than 10 $\mu$m, and more preferably not more than 1 $\mu$m.

The inorganic fine particles before mixed with a water absorbent resin may be in a form of powder or in a form of a water dispersion (slurry (e.g. colloidal silica)). Alternatively, the inorganic fine particles before mixed with a water absorbent resin may be in a form of being dispersed in a surface crosslinking agent or an aqueous solution of the surface crosslinking agent.

(Cationic polymer compound)

The cationic polymer compound is not particularly limited. However, the cationic polymer compound is suitably any of cationic polymer compounds described in U.S. Patent No. 5382610, U.S. Patent No. 7098284, WO2009/110645, WO2009/041731, WO2009/041727. Among the compounds described in the above-listed documents, polyethylene imine, polyvinyl amine, polyallylamine, or a condensate of dimethylamine, ammonia, and epichlorohydrin are preferable as the cationic polymer compound in the present invention.

As for a molecular weight of the cationic polymer compound, a weight average molecular weight is preferably in a range from 1,000 to 5,000,000, more preferably in a range from 2,000 to 1,000,000, and still more preferably in a range from 10,000 to 500,000.

The cationic polymer compound is preferably a water-soluble compound from the viewpoint of facilitating mixture. Here, the term "water-soluble" means that a compound dissolves in an amount of not less than 1g in 100 g of water (25 °C).

The cationic polymer compound may be directly mixed with a water absorbent resin or may be mixed in a form of a solution, particularly in a form of an aqueous solution. Alternatively, the cationic polymer compound may be mixed in a form of being dissolved in a surface crosslinking agent or in an aqueous solution of the surface crosslinking agent.

(Water-soluble polyvalent metal cation-containing compound)

The water-soluble polyvalent metal cation-containing compound refers to a compound containing a bivalent or higher metal cation, preferably a trivalent or higher metal cation. The trivalent or higher metal cation is exemplified by aluminum, zirconium, and titanium. Among these, aluminum is preferable. Examples of the polyvalent metal cation-containing compound encompass (i) polyvalent metal compounds, which are inorganic surface crosslinking agents, including inorganic salts of polyvalent metals such as aluminum sulfate, aluminum chloride, zirconium chloride oxide, zirconium ammonium carbonate, zirconium potassium carbonate, zirconium sulfate, zirconium acetate, and zirconium nitrate, etc.; (ii) polyvalent metal compounds including organic salts of polyvalent metals such as aluminum acetate, aluminum lactate, hydroxy zirconium chloride, titanium triethanol aminate, and titanium lactate, etc.; and the like. Among these, a compound containing aluminum as the polyvalent metal cation is preferable.

These compounds may be directly mixed in a form of a powder with a water absorbent resin. Alternatively, they may be mixed in a form of a solution or a dispersion, particularly in a form of an aqueous solution. Further alternatively, they may be mixed in a form of being dissolved in a surface crosslinking agent or an aqueous solution of the surface crosslinking agent.

The amount of the additive or liquid permeability enhancer selected from the water-insoluble fine particulate compounds and polyvalent cationic compounds is preferably in a range from 0.001 to 5 parts by mass, more preferably in a range from 0.01 to 2 parts by mass, and still more preferably in a range from 0.01 to 1 part by mass, relative to 100 parts by mass of a water absorbent resin to which the additive or liquid permeability enhancer is to be added. It should be noted that in a case where the additive or liquid permeability enhancer is the water-soluble polyvalent metal cation-containing compound, these values are expressed in terms of the amount of polyvalent metal cation.

In a production method according to the present invention, a water-soluble polyvalent metal cation-containing compound may be added twice or more times. For example, in a case where the water-soluble polyvalent metal cation-containing compound is added twice, a ratio between a first addition and a second addition is in a range from 1:99 to 99:1 and preferably in a range from 10:90 to 90:10. A ratio falling outside the above ranges is not preferable because it causes a situation extremely close to one-time addition, which reduces effectiveness of plurality of additions.

It should be noted that a non-metallic ion crosslinking agent such as a cationic polymer compound can express tackiness at the aforementioned mixture. In view of this, the addition of the non-metallic ion crosslinking agent is preferably performed after the last heat treatment.

In a case where a solvent is used for mixture of the water-soluble polyvalent metal cation-containing compound, the solvent is preferably water or an aqueous crosslinking agent solution. For the improvement in dispersity, solubility, and blendability, water may be used in combination with a hydrophilic organic solvent (alcohol or polyglycol) or a surfactant, if necessary. The amount of water used is appropriately determined according to a kind of additive and an addition method and is, for example, in a range from 0 part by mass (dry blending) to 50 parts by mass, in a range from 0.1 part by mass to 10 parts by mass, or in a range from 0.5 part by mass to 5 parts by mass, relative to 100 parts by mass of a water absorbent resin.

Further, as a liquid permeability enhancer other than the above liquid permeability enhancers, a water-soluble polysiloxane described in the pamphlet of International Publication No. WO 2009/093708, primary to tertiary amine compounds described in the pamphlet of International Publication No. WO 2008/108343, etc. can be preferably used.

(Step of adding other additive(s))

The present step is a step of adding other additive(s) in order to impart various functions to a surface-crosslinked water absorbent resin and is composed of one step or a plurality of steps. As the additive, the aforementioned liquid permeability enhancer, a deodorant, a perfume, an antimicrobial agent, a foaming agent, a chelating agent, a surfactant, an anti-coloring agent, a pigment, a dye, a fertilizer, an oxidizing agent, a reducing agent, etc. may be contained for imparting or improving the functions.

These additives are used in such a manner that the proportion of these additives to surface-crosslinked water absorbent resin particles is less than 10 mass%, preferably less than 5 mass%, and more preferably less than 1 mass%. Further, the addition of these additives may be performed simultaneously with or separately from the surface crosslinking step.

[3] Physical properties of polyacrylic acid (salt)-based water absorbent resin powder

(3-1) AAP (Absorption capacity under load)

Surface crosslinking after the polymerization can be attained by, for example, a water absorbent resin powder that shows an absorption capacity (AAP) of not less than 15 (g/g), preferably not less than 17 (g/g), and more preferably not less than 19 (g/g) for a 0.9 wt% of sodium chloride aqueous solution under a load of 4.8 kPa. Higher AAP of the water absorbent resin powder is thus more preferable. However, from the viewpoint of a balance between the AAP and other physical properties (e.g. SFC), an upper limit of the AAP of the water absorbent resin powder is preferably not more than 40 (g/g), more preferably not more than 35 (g/g), and still more preferably not more than 30 (g/g). Note that AAP of a water absorbent resin powder can be controlled by surface crosslinking, CRC, and a liquid permeability enhancer.

(3-2) CRC (Absorption capacity without load)

A water absorbent resin powder in the present invention is a water absorbent resin powder having an absorption capacity without load (CRC) of not less than 20 (g/g), preferably not less than 23 (g/g), and more preferably not less than 25 (g/g). In a case where the absorption capacity without load is low, use in sanitary materials such as diapers may become inefficient in efficiency of water absorption. Higher CRC of the water absorbent resin powder is thus more preferable. However, from the viewpoint of a balance between the CRC and other physical properties (e.g. SFC), an upper limit of the CRC of the water absorbent resin powder is preferably not more than 60 (g/g), more preferably not more than 50 (g/g), and still more preferably not more than 35 (g/g). The CRC of a water absorbent resin powder can be controlled by crosslinking density in polymerization and/or in surface crosslinking.

(3-3) SFC (Saline Flow Conductivity)

An example means of achieving the polymerization and surface-crosslinking of particle-size adjusted particles is a use of a water absorbent resin powder exhibiting the saline flow conductivity (SFC) (liquid permeability of a solution under load) for 0.69 wt% saline of not less than $10(\times 10^{-7} \cdot cm^3 \cdot sec/g)$, more preferably not less than $15(\times 10^{-7} \cdot cm^3 \cdot sec/g)$, still more preferably not less than $30(\times 10^{-7} \cdot cm^3 \cdot sec/g)$.

The saline flow conductivity (SFC) depends on a water absorbent resin composition content (wt%) of a sanitary material. The higher the water absorbent resin composition content is, the higher saline flow conductivity (SFC) is required. From the viewpoint of a balance between the SFC and other physical properties (e.g. CRC), an upper limit of the SFC is approximately not more than $1000(\times 10^{-7} cm^3 \cdot sec/g)$. The SFC can be controlled by (i) the aforementioned particle size, (ii) CRC, and (iii) crosslinking density during polymerization or during surface crosslinking (particularly surface crosslinking).

(3-4) Extr.

Extr. (water soluble component) of the water absorbent resin and the water absorbing agent obtained by the present invention is preferably 5 mass% to 20 mass%, more preferably 5 mass% to 18 mass%, and still more preferably 5 mass% to 15 mass%. In a case where the Extr. exceeds 20 mass%, a gel strength of an obtained water absorbent resin or an obtained water absorbing agent is weak. This may cause inferior liquid permeability. In a case where such a water absorbent resin is used for a water absorbent body such as a diaper, it may not be possible to obtain a water absorbent resin having little amount of rewetting when pressure is applied to the water absorbent body.

Extr. can be controlled as necessary by the aforementioned internal crosslinking agent or the like. Note, however, that a water absorbent resin or a water absorbing agent having an Extr. of less than 5 mass% is not preferable because such a water absorbent resin or water absorbing agent (i) requires a large amount of internal crosslinking agent to be produced, (ii) causes an increase in cost and the occurrence of remaining crosslinking agent beyond the limit of detection, and (iii) causes significant deterioration of the CRC.

(3-5) Particle size and additive for functionalization

There are no limitations on particle diameter and particle size distribution of the water absorbent resin and the water absorbing agent obtained by the present invention. However, it is preferable to obtain, by particle sizing after addition and mixing of a final surface crosslinking agent, a water absorbent resin and a water absorbing agent having particle diameter of less than 1 mm or even the following particle diameter. Assume that a large amount of particles having a diameter of not less than 1 mm, especially not less than 850 μm, is contained in a water absorbent resin and a water absorbing agent. In this case, if such large-size particles are used for especially a thin sanitary material/absorbent article, not only the large-size particles cause uncomfortableness of a user wearing the thin sanitary material/absorbent article, but also a water impermeable material (known as a back sheet) constituting an absorbing product becomes damaged by abrasion and therefore may cause leakage of urine or the like in actual use. Hence, such large-size particles are not preferable. Therefore, particles having not less than 850 μm are preferably little in amount. The particles having diameters of not less than 850 μm are preferably 0 mass% to 5 mass%, more preferably 0 mass% to 3 mass%, still more preferably 0 mass% to 1 mass%, and particularly preferably not contained at all in practice. Furthermore, the amount of contained large-size particles having diameters of not less than 710 μm are preferably 0 mass% to 20 mass%, more preferably 0 mass% to 10 mass%, still more preferably 0 mass% to 5 mass%, further still more preferably 0 mass% to 3 mass%, and particularly preferably 0 mass% to 1 mass%.

On the other hand, a percentage of fine particles having diameters of less than 150 μm is preferably in a range from 0 mass% to 3 mass%, more preferably in a range from 0 mass% to 2 mass%, and still more preferably in a range from 0 mass% to 1.5 mass%.

Furthermore, while maintaining the above ranges, particles having diameters of not less than 150 μm and less than 850 μm, more preferably not less than 150 μm and less than 710 μm, are preferably contained to be not less than 95 mass%, more preferably not less than 98 mass%, still more preferably not less than 99 mass%, and most preferably contained in their entirety in practice.

A mass average particle diameter (defined by standard sieve classification) of water absorbent resin particles of a water absorbing agent to be obtained as a final product after the above steps in the present invention is preferably not less than 200 μm and not more than 600 μm, more preferably in the range of 550 μm to 200 μm in order to improve its performance, still more preferably in the range of 500 μm to 250 μm, and most preferably in the range of 450 μm to 350 μm. A percentage of particles having particle diameters of less than 300 μm is preferably not less than 10 mass%, more preferably in the range of 10 mass% to 50 mass%, and still more preferably in the range of 10 mass% to 30 mass%.

Particle size can be controlled as necessary by pulverizing, classification (pre-surface crosslinking and post-surface crosslinking), granulation, and the like.

In a case where the above ranges are not maintained, it may not be possible to obtain a balanced water absorbent resin having excellent liquid permeability while maintaining desired absorption capacity. Particularly, particles having particle diameters of less than 150 μm are preferably as little as possible in amount because such particles not only cause a reduction in liquid permeability, but also may cause adverse effect by generating dust etc. in an environment where an absorbent article as a material for a water absorbent resin is produced.

The water absorbing agent according to the present invention preferably contains, other than surface-crosslinked water absorbent resin particles, (i) a liquid permeability enhancer or (ii) an additive selected from water-insoluble fine particulate compounds and polyvalent cationic compounds. Furthermore, the water absorbing agent of the present invention can have imparted or increased functionality by containing additives such as a deodorant, a perfume, an antimicrobial agent, a foaming agent, a chelating agent, a surfactant, an anti-coloring agent, a pigment, a dye, a fertilizer, an oxidizing agent, and a reducing agent. A percentage of such additives used relative to a total amount of water absorbent resin particles and water-soluble polyvalent metal salt particles is less than 10 mass%, preferably less than 5 mass%, and more preferably less than 1 mass%.

[4] Use or the like of particulate water absorbing agent

The water absorbent resin of the present invention is to be used for sanitary materials such as disposable diapers, sanitary napkins, incontinence pads, and medical pads. In a case where the water absorbent resin of the present invention is used for a sanitary material, the sanitary material to be used preferably includes (a) a liquid permeable top sheet positioned next to a body of a user, (b) a liquid-impermeable back sheet positioned away from the body of the user and positioned next to clothing of the user, and (c) a water absorbent body positioned between the top sheet and the back sheet. The water absorbent body can be made up of two or more layers, or can be used in combination with a pulp layer.

In the case where the water absorbent resin of the present invention is used for a sanitary material, a gel having absorbed liquid is unlikely to cause so-called gel blocking, and therefore spaces between gels are not blocked as a result of the gels in close contact with each other. Hence, even in a case where the water absorbent resin is used at high concentration in an absorbent body such as a diaper, urine and bodily fluids discharged for second or subsequent times can be spread out inside the absorbent body without being stuck on a surface of the absorbent body. This allows the urine and the bodily fluids to be distributed throughout the water absorbent resin inside the absorbent body.

Examples

The following description will discuss the invention with reference to Examples, Comparative Examples, and Reference Examples. However, interpretation of the present invention should not be limited by the examples or the like. Physical properties described in the Claims and the Examples of the present invention except for (5-7) Moisture absorption blocking rate were calculated at room temperature ($23\pm2$ °C) and at a humidity of $50\pm10$ RH%. Further, physical properties described in the Claims and the Examples of the present invention were calculated by measurement methods in Sections (5-1) to (5-8) below. Note that unless specified otherwise, each step in each example was carried out under substantially atmospheric pressure (atmospheric pressure $\pm5$%, more preferably $\pm1$%), and was carried out without changing pressure by intentionally increasing or decreasing the pressure in the same step.

(5-1) Absorption capacity without load (CRC)

In accordance with ERT441.2-0.2, 0.200 g of water absorbent resin was allowed to freely swell in a large excess of a 0.90 wt% of sodium chloride aqueous solution (also referred to as "physiological saline") without load for 30 minutes. Then, an absorption capacity (CRC) after water was drained by centrifugal separation was measured.

(5-2) Absorption capacity under load (AAP/Absorbency Against Pressure)

In accordance with the absorption capacity under load evaluation method disclosed in the publication of EDANA (European Disposables and Nonwovens Association) and the method described in ERT442.2-02, a one-hour measurement was carried out with use of 0.900 g of water absorbent resin and a 0.9 mass% of sodium chloride aqueous solution, and absorption capacity under load (g/g) of water absorbent resin was calculated under such excessive load as 4.83 kPa (approximately 0.7 psi).

(5-3) Liquid permeability (SFC)

SFC is a well known measurement method and was measured by the method which is disclosed in U.S. Patent No. 5562646.

(5-4) Particle size distribution (PSD) (ERT420.2-02)

The term "PSD" stands for Particle Size Distribution, and means a particle size distribution measured by sieve classification. It should be noted that mass average particle diameter (D50) and the particle diameter distribution width were calculated based on the result of measurement performed in the same manner as in the "(1) Average Particle Diameter and Distribution of Particle Diameter" described in U.S. Patent No. 2006/204755.

(5-5) Extr

Measurement was carried out in accordance with the water soluble component evaluation method disclosed in the publication of EDANA (European Disposables and Nonwovens Association) and the method described in ERT470.2-02.
Specifically, 200 g of 0.90 mass% saline was measured and put in a plastic container of 250 ml in capacity with a lid, thereby obtaining an aqueous solution. Then, 1.00 g of water absorbent resin particles or water absorbing agent was added to the aqueous solution, thereby obtaining a mixture. Then, the mixture was stirred at $500\pm50$ rpm by use of a stirrer (length: 3.5 cm) for 16 hours, thereby extracting soluble component in the resin (mainly soluble polyacrylate and the like).
This extract was filtered by use of a sheet of filter paper (manufactured by ADVANTEC TOYO KAISHA LTD., Product name: (JIS P 3801, No.2), thickness: 0.26 mm, retaining particle diameter: 5 $\mu$m), thereby obtaining a filtrate. 50.0 g of the filtrate was measured so that a measurem.ent solution was prepared.
First, 0.90 mass% saline was titrated with the use of 0.1 N NaOH aqueous solution until it reached pH10. Then, the saline was titrated with the use of 0.1 N HCl aqueous solution until it reached pH2.7, thereby obtaining control titres ([bNaOH] ml, [bHCl] ml).
The measurement solution was also titrated in the same manner, thereby obtaining titres (NaOH] ml, [HCl] ml).
In a case of, for example, water absorbent resin particles or water absorbing agent which include(s) a known amount of acrylic acid and sodium salt, soluble component of the water absorbent resin particles or of the water absorbing agent can be calculated (i) based on an average molecular weight of monomers and on titres obtained by the aforementioned titration and (ii) by the following equation:

$$\text{soluble component (mass\%)} = 0.1 \times (\text{average molecular weight}) \times 200 \times 100 \times ([\text{HCl}] - [\text{bHCl}])/1000/1.0/50.0$$

In a case where the amount of acrylic acid and sodium salt is unknown, an average molecular weight of monomers is calculated with a neutralization rate obtained by titration.

(5-6) Residual surface crosslinking agent and residual byproduct

Measurements on a residual surface crosslinking agent and a residual byproduct were made in the following manner. That is, 1 g of water absorbent resin was allowed to swell in a 0.9 wt% of sodium chloride aqueous solution (saline). After 1 hour stirring, a resultant solution was subjected to filtration with use of a 0.45-μm disc filter. A filtrate thus obtained was subjected to measurement by high-performance liquid chromatography (HPLC) so that the residual amounts relative to the weight of a water absorbent resin were measured. It should be noted that detection limit (N.D. level) was not more than 100 ppm.

(5-7) Moisture absorption blocking rate

The moisture absorption blocking rate was determined by performing measurement in the following manner. That is, 2.0 g of water absorbent resin or water absorbing agent was allowed to stand for 30 minutes in a thermo-hygrostat set under such a condition that atmospheric temperature was 25 °C and relative humidity was 70 %RH, after which clusters obtained after moisture absorption and aggregation were measured in mass. It can be said that the smaller a value of the blocking rate after moisture absorption is, the higher the anti-caking property is.

Specifically, the condition was as follows. That is, the thermo-hygrostat used was THN040FA (manufactured by ADVANTEC) which enables adjustment of atmospheric temperature to $25 \pm 0.5$ °C and adjustment of relative humidity to $70 \pm 3$ %RH, and an aluminum cup of 52 mm in bottom diameter and 2.5 mm in height was used as a container for 2.0g of water absorbent resin or water absorbing agent. The 2.0 g of sample was weighed out into the container and was then allowed to stand for 30 minutes in the thermo-hygrostat previously set to the condition as described above.

After being allowed to stand for 30 minutes, the container was turned upside down on a 2-mm JIS standard sieve (including a saucer), and a water absorbent resin or a water absorbing agent taken out of the container was transferred onto the sieve. It should be noted that by measuring the mass of the aluminum cup before the measurement, the amount ($W_{adh}$) of a sample after moisture absorption which sample was not fallen on the sieve even when the container was turned upside down was recorded as the amount of aggregates. The 2-mm sieve was tapped 30 times so that the sample after moisture absorption, which sample had been fallen onto the 2-mm sieve, was classified into (i) clusters obtained after moisture absorption and aggregation and (ii) particles causing no aggregation. In this manner, the mass of aggregates of not less than 2 mm and the mass of particles of less than 2 mm ($W_{on}$ and $W_{pass}$, respectively) were measured. Moisture absorption blocking rate (%) was calculated by the following equation:

$$\text{Moisture absorption blocking rate (\%)} = (W_{adh} + W_{on})/(W_{adh} + W_{on} + W_{pass}) \times 100.$$

(5-8) Free swelling capacity (FSC)

In accordance with ERT440.2-02, free swelling capacity (FSC) relative to 0.9% saline was measured. It should be noted that FSC is different from CRC (ERT441.2-0.2) described above in that draining performed after water absorption is made under suspension rather than by means of centrifugation.

(Reference Example 1)

A solution (A) was prepared by mixing 421.7 g of acrylic acid, 3.06 g of polyethyleneglycol diacrylate (weight average molecular weight: 523, the polyethyleneglycol diacrylate as an internal crosslinking agent is such that the average number (n) of moles of ethylene oxide added is 9) (0.10 mol%), and 1.29 g of a 2 mass% of diethylentriamine pentaacetate trisodium aqueous solution (manufactured by CHELEST CORPORATION). A NaOH aqueous solution (B) was prepared by diluting 352.3 g of a 48.5 mass% of NaOH aqueous solution with 402.7 g of ion exchange water.

While the solution (A) was stirred with a magnetic stirrer, the aqueous solution (B) was added to the solution (A) all at once in an open system, and was mixed. Although an educt was observed at the beginning of the mixing, the educt was immediately dissolved, thereby obtaining a monomer aqueous solution (monomer concentration: 43 mass%, neutralization rate: 73 mol%). Then, to the monomer aqueous solution, 19.4 g of a 3.6 mass% of sodium persulfate aqueous solution was added. A resultant solution was stirred for several seconds, and immediately placed on a hot plate set to a temperature of 90 °C. Then, in an open system, the solution was poured into a stainless vat having an inner surface to which a silicon sheet was attached. The stainless vat has a bottom surface measuring 200 mm×260 mm, a top surface measuring 560 mm×460 mm, and a height of 140 mm. A vertical cross section of the stainless vat has a trapezoidal shape when passing through a center part of the stainless vat. Polymerization was initiated while the top surface of the stainless vat was opened. The polymerization proceeded while the solution was generating water vapors, foaming, and expanding, so that a hydrous polymer was generated. After the polymerization, the hydrous polymer was taken out from the stainless vat.

The hydrous polymer was divided equally into 16 pieces. Then, the pieces of the hydrous polymer were crushed by introducing the pieces, one piece for every 10 seconds, into a meat chopper (manufactured by REMACOM HL-3225N) having a dice of 9.5 mmΦ while 50 g of ion exchange water was added each minute. The hydrous polymer, which had been crushed and gained refining, was spread on a 50-mesh metal net (having mesh size of 300 μm), and was dried by hot-air drying at a temperature of 190 °C for 50 minutes. In this manner, a particulate or powdery irregular-shaped water absorbent resin that can be easily pulverized or a water absorbent resin in the form of particulate dry aggregates was obtained.

The water absorbent resin thus obtained was pulverized with a roll mill, and classified with the use of a JIS standard sieve having mesh size of 710 μm. Particles remaining on the sieve were removed. Next, as in the aforementioned operations, particles passing through the JIS standard sieve having mesh size of 710 μm were classified with the use of a JIS standard sieve having mesh size of 150 μm, thereby obtaining a water absorbent resin (F1) passing through the JIS standard sieve having mesh size of 150 μm and a particulate water absorbent resin (1). CRC and soluble component of the water absorbent resin (1) thus obtained were 33.0 g/g and 8.5%, respectively. Particle size distribution of the water absorbent resin (1) thus obtained is shown in Table 3.

(Example 1)

Relative to 100 parts by mass of the water absorbent resin (1) obtained in Reference Example 1, 4.1 parts by mass of surface treatment agent mixture solution containing 2-oxo-1,3-dioxolane, 1,2-propanediol, and ion exchange water (in a mixture ratio (mass ratio) of 0.4:0.7:3.0) was added and mixed. In the mixing, a Loedige mixer (manufactured by Gerbrueder Ledige Maschibenbau GmbH) was used as a mixer. The water absorbent resin (1) and a surface treatment agent mixture solution were mixed together by spraying the surface treatment agent mixture solution onto the water absorbent resin (1) with the use of a spray nozzle (single-fluid hollow cone nozzle (1/4M-K-008) manufactured by H. IKEUCHI Co., Ltd.). A resultant mixture was evenly spread on a SUS vat. The SUS vat was allowed to stand in a drying apparatus in which an atmospheric temperature and a dew point were conditioned to be at 197 °C and -5 °C, respectively, as measured by Humidity and Temperature Transmitter HMT337, Serial No. G1110105 (manufactured by VAISALA). Then, a heat treatment was carried out for 25 minutes. Particles after the heat treatment were allowed to pass through a JIS standard sieve having mesh size of 710 μm, thereby obtaining a surface-crosslinked water absorbent resin (1) in which the periphery of the surface thereof is cross-linked.

Relative to 100 parts by mass of the surface-crosslinked water absorbent resin (1) thus obtained, 1.2 parts by mass of a mixture solution was added, which mixture solution contains a 27 mass% of aluminum sulfate aqueous solution (8 mass% based on aluminum oxide), a 60 mass% of sodium lactate aqueous solution, and 1,2-propylene glycol (in a mixture ratio (mass ratio) of 1:0.3:0.025). After the addition, a resultant mixture was dried at 60 °C for 1 hour with no air flow. Next, resultant particles were allowed to pass through a JIS standard sieve having mesh size of 710 μm, thereby obtaining a water absorbing agent (1). Physical properties and particle size distribution of the water absorbing agent (1) thus obtained are shown in Tables 1 and 3, respectively.

(Example 2)

Exactly the same operations as in Example 1 were carried out except that the dew point at the heat treatment was changed to 40 °C, that the heat treatment time was extended to 30 minutes, and that the particles passing through the 710-μm sieve were classified with use of a JIS standard sieve having mesh size of 150 μm. In this manner, a water absorbent resin (F2) passing through the JIS standard sieve having mesh size of 150 μm, a surface crosslinked water absorbent resin (2), and a water absorbing agent (2) were obtained. As for the water absorbing agent (2) thus obtained, its physical properties and particle size distribution are shown in Tables 1 and 3, respectively.

(Comparative Example 1)

Surface crosslinking was carried out in accordance with Patent Literature 38 (International Publication No. WO 2009/125849). That is, exactly the same operations as in Example 1 were carried out except that the dew point at the heat treatment was changed to 80 °C, and that the heat treatment time was extended to 35 minutes. In this manner, a comparison-use surface crosslinked water absorbent resin (1) and a comparison-use water absorbing agent (1) were obtained. As for the comparison-use water absorbing agent (1) thus obtained, its physical properties and particle size distribution are shown in Tables 1 and 3, respectively.

(Comparative Example 2)

Surface crosslinking was carried out in accordance with Patent Literature 38. That is, exactly the same operations as in Example 1 were carried out except that the dew point at the heat treatment was changed to 90 °C and that the heat treatment time was extended to 40 minutes. In this manner, a comparison-use surface crosslinked water absorbent resin (2) and a comparison-use water absorbing agent (2) were obtained. As for the comparison-use water absorbing agent (2) thus obtained, its physical properties and particle size distribution are shown in Tables 1 and 3, respectively.

(Comparative Example 3)

The surface crosslinked water absorbent resin (1) obtained in Example 1 was used directly as a comparison water absorbing agent (3).

(Comparative Example 4)

The surface crosslinked water absorbent resin (2) obtained in Example 2 was used directly as a comparison water absorbing agent (4).

(Reference Example 2)

Exactly the same operations as in Reference Example 1 were carried out except that the amount of polyethyleneglycol diacrylate was changed from 3.06 g to 0.90 g (0.03 mol%) and that mesh size of the sieve for classifying the particles passing through the JIS standard sieve having mesh size of 710 $\mu$m after the pulverization with use of a roll mill was changed to 75 $\mu$m. In this manner, a water absorbent resin (2) was obtained. As for the water absorbent resin (2) thus obtained, CRC was 47.0 g/g, and soluble component was 16.1 %. Particle size distribution of the water absorbent resin (2) thus obtained is shown in Table 3.

(Comparative Example 5)

Surface crosslinking was carried out in accordance with Example 7 of Patent Literature 36 (European Patent No. 119051).
That is, relative to 100 parts by mass of the water absorbent resin (2) obtained in Reference Example 2, 4.5 parts by mass of a surface treatment agent mixture solution containing 1,3-propanediol, propylene glycol, ion exchange water, and ethanol (in a mixture ratio (mass ratio) of 0.5:0.5:3.0:0.5) was added and mixed as in Example 1. A resultant mixture was subjected to heat treatment for 60 minutes while being stirred with use of a paddle type drier to which adjustments were made such that an inner wall (heat medium) temperature was 185 °C, an atmospheric temperature measured by Humidity and Temperature Transmitter described in Example 1 was 97 °C, and a dew point was 40 °C. In this manner, a comparison-use surface crosslinked water absorbent resin (5) was obtained. As for the comparison-use surface crosslinked water absorbent resin (5) thus obtained, its physical properties and particle size distribution are shown in Tables 1 and 3, respectively.

(Example 3)

The same operations as in Example 1 were carried out except that the drier used for the surface crosslinking in Example 1 was changed to the paddle type drier described in Comparative Example 5, that adjustments were made such that the inner wall (heat medium) temperature was changed to 198 °C, the atmospheric temperature was changed to 105 °C, and the dew point was changed to 40 °C and that the heat treatment time was extended to 30 minutes. In this manner, surface crosslinking was made until the CRC reached approximately 27.5 g/g so that a water absorbing agent (3) was obtained. As for the water absorbing agent (3) thus obtained, its physical properties and particle size distribution are shown in Tables 1 and 3, respectively.

(Example 4)

The same operations as in Example 1 were carried out except that 2-oxo-1,3-dioxolane used as the surface crosslinking agent solution in the surface treatment agent mixture solution in Example 1 was replaced by 1,3-propanediol so that a surface treatment agent mixture solution containing of 1,3-propanediol, 1,2-propanediol, and ion exchange water (in a mixture ratio (mass ratio) of 0.3:0.7:3.0) was used, and that the atmospheric temperature was adjusted to 210 °C. In this manner, a water absorbing agent (4) was obtained. Physical properties of the water absorbing agent (4) thus obtained are shown in Table 1.

(Example 5)

The same operations as in Example 1 were carried out except that 2-oxo-1,3-dioxolane used as the surface crosslinking agent solution in the surface treatment agent mixture solution in Example 1 was replaced by 1,4-butanediol so that a surface treatment agent mixture solution containing of 1,4-butanediol, 1,2-propanediol, and ion exchange water (in a mixture ratio (mass ratio) of 0.4:0.7:3.0) is used, and that the atmospheric temperature was adjusted to 210 °C. In this manner, a water absorbing agent (5) was obtained. Physical properties of the water absorbing agent (5) thus obtained are shown in Table 1.

(Comparative Example 6)

The same operations as in Example 3 were carried out except that the dew point in Example 3 was changed to 80 °C and that the heat treatment time was extended to 40 minutes. In this manner, surface crosslinking was made until the CRC reached approximately 27.5 g/g so that a comparison water absorbing agent (6) was obtained. Physical properties of the comparison water absorbing agent (6) thus obtained are shown in Table 1.

(Reference Example 3)

In a reactor formed by attaching a lid to a double-arm type stainless kneader having a capacity of 10 liters and equipped with two sigma type blades and a jacket, 436.4g of acrylic acid, 4617.9g of a 37 mass% of sodium acrylate aqueous solution, 395.96g of ion exchange water, 10.13 g (0.08 mol%) of polyethyleneglycol diacrylate (molecular weight: 523, the polyethyleneglycol diacrylate as an internal crosslinking agent is such that the average number (n) of moles of ethylene oxide added is 9), and 0.873g of (0.04mol%) 1,4-butanediol were dissolved so that a reaction solution was prepared. Further, the reaction solution was degassed in a nitrogen gas atmosphere for 20 minutes while the temperature of the reaction solution was maintained at 25 °C. Dissolved oxygen in the reactor was not more than 1 ppm.
Subsequently, 14.53 g of a 20 mass% of sodium persulfate aqueous solution and 24.22 g of a 0.1 mass% of L-ascorbic acid aqueous solution were added to the reaction solution with stirring. About 34 seconds after the addition, the reaction solution reached 25.5 °C, and polymerization thereof started. Then, a generated gel was polymerized at a temperature in a range from not lower than 25.5 °C to not higher than 92 °C while being crushed. After 30 minutes after the start of polymerization, the generated hydrous polymer was taken out.
The hydrous polymer thus obtained was a polymer that had been gained refining and had a particle diameter of about not more than 5 mm. The hydrous polymer thus gained refining was spread on a 50-mesh metal net (having mesh size of 300 $\mu$m) and then hot-air dried at 180 °C for 45 minutes.
In this manner, a particulate or powdery irregular-shaped water absorbent resin that can be easily pulverized or an irregular-shaped water absorbent resin in the form of particulate dry aggregates that can be easily pulverized was obtained. The water absorbent resin thus obtained was pulverized with use of a roll mill and then classified with use of a JIS standard sieve having mesh size of 710 $\mu$m. Particles remaining on the sieve were removed. Next, as in the aforementioned operations, particles passing through the JIS standard sieve having mesh size of 710 $\mu$m were classified with use of a JIS standard sieve having mesh size of 150 $\mu$m in order to remove a water absorbent resin passing through the JIS standard sieve having mesh size of 150 $\mu$m. In this manner, a particulate water absorbent resin (3) was obtained. The CRC of the water absorbent resin (3) thus obtained was 32.1 g/g.

(Example 6)

The same operations as in Example 1 were carried out except that the water absorbent resin (1) used in Example 1 was replaced by the water absorbent resin (3) obtained in Reference Example 3, that the dew point and heat treatment time at the heat treatment in the step of obtaining the surface-crosslinked water absorbent resin (1) were changed to 35 °C and to 30 minutes, respectively. In this manner, a surface-crosslinked water absorbent resin (7) was obtained.

Relative to 100 parts by mass of the surface-crosslinked water absorbent resin (7) thus obtained, 4 parts by mass of a mixture solution in which (i) a dimethylamine-ammonium-epichlorohydrin resin aqueous solution (manufactured by SENKA Corporation, UNISENCE KHE102L that is an aqueous solution having average molecular weight of approximately 70,000, 1% aqueous solution pH of approximately 6, and solid content concentration of 50 mass%) and (ii) methanol were mixed with each other (in a mixture ratio (mass ratio) of 1:1) was added. After the addition, resultant particles were dried at 90 °C for 1 hour with no air flow. Subsequently, the particles obtained were sieved with a JIS standard sieve having mesh size of 850 $\mu$m. In this manner, a water absorbing agent (6) was obtained. Physical properties of the water absorbing agent (6) thus obtained are shown in Table 1.

(Comparative Example 7)

The same operations as in Example 6 were carried out except that the dew point at the heat treatment was changed to 90 °C and that the heat treatment time was extended to 50 minutes. In this manner, a comparison-use surface crosslinked water absorbent resin (7) and a comparison-use water absorbing agent (7) were obtained. Physical properties of the comparison-use water absorbing agent (7) thus obtained is shown in Table 1.

(Example 7)

The same operations as in Example 1 were carried out except that the surface treatment agent mixture solution used in Example 1 was replaced by 3.95 parts by mass of a mixture solution containing ethylene glycol, 1,2-propanediol, and water (in a mixture ratio (mass ratio) of 0.25:0.7:3.0), that the atmospheric dew point at the heat treatment was changed to 35 °C, and that the heating time for the surface crosslinking treatment was changed to 45 minutes. In this manner, a water absorbing agent (7) was obtained. Physical properties of the water absorbing agent (7) thus obtained are shown in Table 2.

(Example 8)

The same operations as in Example 1 were carried out except that the surface treatment agent mixture solution used in Example 1 was replaced by 4.15 parts by mass of a mixture solution containing diethylene glycol, 1,2-propanediol, and water (in a mixture ratio (mass ratio) of 0.45:0.7:3.0), that the atmospheric dew point at the heat treatment was changed to 35 °C, and that the heating time for the surface crosslinking treatment was changed to 35 minutes. In this manner, a water absorbing agent (8) was obtained. Physical properties of the water absorbing agent (8) thus obtained are shown in Table 2.

(Example 9)

The same operations as in Example 1 were carried out except that the surface treatment agent mixture solution used in Example 1 was replaced by 4.1 parts by mass of a mixture solution containing 1,2- propylene carbonate, 1,2-propanediol, and water (in a mixture ratio (mass ratio) of 0.4:0.7:3.0), that the atmospheric dew point at the heat treatment was changed to 35 °C, and that the heating time for the surface crosslinking treatment was changed to 40 minutes. In this manner, a water absorbing agent (9) was obtained. Physical properties of the water absorbing agent (9) thus obtained are shown in Table 2.

(Example 10)

The same operations as in Example 1 were carried out except that the surface treatment agent mixture solution used in Example 1 was replaced by 4.05 parts by mass of a mixture solution containing 1,3-butanediol, 1,2-propanediol, and water (in a mixture ratio (mass ratio) of 0.35:0.7:3.0), that the atmospheric dew point at the heat treatment was changed to 35 °C, and that the heating time for the surface crosslinking treatment was changed to 40 minutes. In this manner, a water absorbing agent (10) was obtained. Physical properties of the water absorbing agent (10) thus obtained are shown in Table 2.

(Reference Example 4)

In a reactor formed by attaching a lid to a double-arm type stainless kneader having a capacity of 10 liters and equipped with two sigma type blades and a jacket, 11.9 g of (0.1 mol%) polyethyleneglycol diacrylate was dissolved in 5432.0 g of an aqueous solution of sodium acrylate (39 mass% in monomer concentration) having neutralization rate of 73 mol% so that a reaction solution was prepared. Next, the reaction solution was degassed in a nitrogen gas atmosphere for 30 minutes. Subsequently, 29.36 g of a 10 mass% of sodium persulfate aqueous solution and 24.47 g of a 0.1 mass% of L-ascorbic acid aqueous solution were added to the reaction solution with stirring. About one minute after the addition, polymerization started. Then, a generated gel was polymerized at a temperature in a range from 20 °C to 95 °C while being crushed. After 30 minutes after the start of polymerization, the generated hydrogel crosslinked polymer was taken out.

The hydrogel crosslinked polymer thus obtained was a polymer that had been gained refining and had a particle diameter of about not more than 5 mm. The hydrogel crosslinked polymer thus gained refining was spread on a 50-mesh metal net (having mesh size of 300 pm) and then hot-air dried at 180 °C for 50 minutes. A water absorbent resin obtained with use of a roll mill was pulverized and further classified with use of JIS standard sieves having respective mesh sizes of 600 $\mu$m and 300 $\mu$m for adjustment of particle size distribution. In this manner, a water absorbent resin (4) was obtained. The water absorbent resin (4) thus obtained had CRC of 35.2 g/g, a soluble component of 8.5 wt%, and a mass average particle diameter of 450 $\mu$m.

(Example 11)

The same operations as in Example 1 were carried out except that the water absorbent resin (1) used in Example 1 was replaced by the water absorbent resin (4), that the surface treatment agent mixture solution was replaced by 3.64 parts by mass of a mixture solution containing ethyleneglycol diglycidyl ether, 1,3-propanediol, and water (in a mixture ratio (mass ratio) of 0.04:1.0:2.6), that the atmospheric dew point at the heat treatment was changed to 35 °C, that the heating time for the surface crosslinking treatment was changed to 30 minutes, and that the sieve used in Example 1 after the addition of the mixture solution in which a 27 mass% of aluminum sulfate aqueous solution, a 60 mass% of sodium lactate aqueous solution, and 1,2-propylene glycol were contained (in a mixture ratio (mass ratio) of 1:0.3:0.025) was changed to a JIS standard sieve having mesh opening size of 600 $\mu$m. In this manner, a water absorbing agent (11) was obtained. Physical properties of the water absorbing agent (11) thus obtained are shown in Table 2.

(Comparative Example 8)

The same operations as in Example 7 were carried out except that the dew point was changed from 35 °C to 80 °C and that the heat treatment time was changed from 35 minutes to 40 minutes. In this manner, a comparison-use water absorbing agent (8) was obtained. Physical properties of the comparison-use water absorbing agent (8) thus obtained is shown in Table 2.

(Example 12)

Exactly the same operations as in Example 11 were carried out except that the surface treatment agent mixture solution used in Example 11 was replaced by 3.60 parts by mass of a mixture solution containing 1,3-propanediol and water (in a mixture ratio (mass ratio) of 1.0:2.6) and that the heating time for the surface crosslinking treatment was changed to 40 minutes. In this manner, a water absorbing agent (12) was obtained. Physical properties of the water absorbing agent (12) thus obtained are shown in Table 2.

(Comparative Example 9)

The same operations as in Comparative Example 8 were carried out except that the dew point was changed from 35 °C to 80 °C and that the heat treatment time was changed from 40 minutes to 50 minutes. In this manner, a comparison-use water absorbing agent (9) was obtained. Physical properties of the comparison-use water absorbing agent (9) thus obtained is shown in Table 2.

(Reference Example 5)

A 2-liter four-necked separable flask was made equipped with a reflux condenser, a dropping funnel, and a nitrogen gas inlet tube, and a 500 mL n-heptane was measured out into the flask. To the flask, 0.92g of sucrose stearic acid ester (S-370, manufactured by Mitsubishi Foods Corporation) (surfactant) was added. A resultant surfactant was heated to 80 °C and then cooled to 35 °C.

Meanwhile, 92.0 g of a 80.5 mass% of acrylic acid aqueous solution was measured out into a 500mL Erlenmeyer flask. While the Erlenmeyer flask was immersed in an ice bath so as to be cooled from the outside, 153.0 g of a 20.0 mass% of sodium hydroxide aqueous solution was added dropwise to the Erlenmeyer flask for neutralization of the acrylic acid to 75 mol%. Thereafter, the mixture was stirred at room temperature to dissolve completely. To the Erlenmeyer flask, 0.11 g of ammonium persulfate and 0.47 g of polyethyleneglycol diacrylate (molecular weight: 523, the average number (n) of moles of ethylene oxide added is 9) were added, and the mixture was then dissolved so that a first-stage monomer aqueous solution was prepared.

With the stirrer being set to 196 rpm in rotational speed, the monomer aqueous solution was added to the separable flask. The separable flask was held at 35 °C for 30 minutes while the system of the separable flask was replaced with nitrogen. Thereafter, the separable flask was immersed in a hot-water bath of 70 °C for temperature rise so that the polymerization was carried out, thereby obtaining a first-stage post-polymerization slurry.

Meanwhile, 143.2 g of a 80.5 mass% of acrylic acid aqueous solution was measured out into another 500mL Erlenmeyer flask. While the Erlenmeyer flask was immersed in an ice bath so as to be cooled from the outside, 239.9 g of a 20.0 mass% of sodium hydroxide aqueous solution was added dropwise to the Erlenmeyer flask for neutralization of the acrylic acid to 75 mol%. Thereafter, the mixture was stirred at room temperature to dissolve completely. To the Erlenmeyer flask, 0.11 g of ammonium persulfate and 0.74 g of polyethyleneglycol diacrylate were added, and the mixture was then dissolved so that a second-stage monomer aqueous solution was prepared.

After the number of stirring rotations for the post-polymerization slurry was changed to 480 rpm, the post-polymerization slurry was cooled to a temperature in a range from 26 °C to 30 °C. The second-stage monomer aqueous solution was added to the system of the separable flask. The separable flask was held for 30 minutes while being replaced with nitrogen. Thereafter, the separable flask was immersed once again in a hot-water bath of 70 °C for temperature rise so that the polymerization was carried out, thereby obtaining a second-stage post-polymerization slurry.

Subsequently, the separable flask was raised in temperature with use of an oil bath of 120 °C, and water and n-heptane were subjected to azeotropy to remove 255.0 g of water out of the system while refluxing n-heptane, after which the separable flask was held at 100 °C for 2 hours. Thereafter, evaporation of n-heptane and drying were carried out to thereby obtain 220.0 g of a water absorbent resin in the form of second particles which are aggregates of spherical primary particles. The water absorbent resin thus obtained had a mass average particle diameter of 400 $\mu$m and a moisture content of 6 mass%. The water absorbent resin thus obtained was classified with use of JIS standard sieves having respective mesh sizes of 600 $\mu$m and 300 $\mu$m for adjustment of particle size distribution. In this manner, a water absorbent resin (5) was obtained. The water absorbent resin (5) thus obtained had CRC of 35.2 g/g, a soluble component of 24.2 wt%, and a mass average particle diameter of 455 $\mu$m.

(Example 13)

The same operations as in Example 12 were carried out except that the water absorbent resin (4) used in Example 12 was replaced by the water absorbent resin (5), that the surface treatment agent mixture solution was replaced by 3.50 parts by mass of a mixture solution containing 1,4butanediol, propylene glycol, and water (in a mixture ratio (mass ratio) of 0.3:0.5:2.7), that the heating temperature was changed to 180 °C, and that the heating time for the surface crosslinking treatment was changed to 45 minutes. In this manner, a water absorbing agent (14) was obtained. Physical properties of the water absorbing agent (14) thus obtained are shown in Table 2.

(Comparative Example 10)

The same operations as in Example 13 were carried out except that the dew point was changed to 80 °C and that the heat treatment time was changed to 55 minutes. In this manner, a comparison-use water absorbingt agent (10) was obtained. Physical properties of the comparison-use water absorbingt agent (10) thus obtained is shown in Table 2.

(Reference Example 6)

In a polymerization container (Dewar bottle) having thermal insulation effectiveness, 155.0 g of acrylic acid, 0.81 g of triallyl isocyanurate (0.15 mol%), and 494.0 g of deionized water were held at 3 °C while being stirred and mixed with each other. After nitrogen was introduced into a resultant mixture so that dissolved oxygen content of the mixture was set to not more than 1 ppm, 15.5g of a 1% of hydrogen peroxide aqueous solution, 1.94 g of a 2% of ascorbic acid aqueous solution, and 23.23 g of a 2% of 2,2'-azobis [2-methyl-propionamide]dihydrochloride aqueous solution were added and mixed, and polymerization was started. After the temperature of the mixture reached 67 °C, polymerization was carried out for about 5 hours at 65 °C, thereby obtaining a hydrogel.

Next, 500 g of the hydrogel was mixed with 90.1 g of a 48.5 % of sodium hydroxide aqueous solution while being crushed by using a double-arm type stainless kneader having a capacity of 2.5 liters and equipped with two sigma type blades and a jacket, to thereby obtain a crushed gel. Further, the crushed gel was dried for 65 minutes by using a ventilation type band dryer (160 °C, air velocity of 2 m/sec) to thereby obtain a water absorbent resin. The water absorbent resin thus obtained was pulverized with use of a roll mill and then classified with use of JIS standard sieves having respective mesh sizes of 850 μm and 150 μm. In this manner, a water absorbent resin (6) was obtained. The water absorbent resin (6) thus obtained had CRC of 34.8 g/g, a soluble component of 8.4 wt%, and a mass average particle diameter of 420 μm.

(Example 14)

The same operations as in Example 13 were carried out except that the water absorbent resin (5) used in Example 13 was replaced by the water absorbent resin (6) and that the heating time for the surface crosslinking treatment was changed to 35 minutes. In this manner, a water absorbing agent (14) was obtained. Physical properties of the water absorbing agent (14) are shown in Table 2.

(Comparative Example 11)

The same operations as in Example 14 were carried out except that the dew point in Example 14 was changed from 35 °C to 80 °C and that the heat treatment time was changed to 45 minutes. In this manner, a comparison-use water absorbing agent (11) was obtained. Physical properties of the comparison-use water absorbing agent (11) are shown in Table 2.

(Reference Example 7)

Relative to 100 parts by mass of the water absorbent resin (F1), which was obtained in Reference Example 1, passing through the JIS standard sieve having mesh size of 150 μm was mixed with 20 parts by mass of the water absorbent resin (F2), which was obtained in Example 2, passing through the JIS standard sieve having mesh size of 150 μm, and a resultant mixture was then subjected to granulation in accordance with a method of Granulation Example 1 described in U.S. Patent No. 6228930. While being mixed, 200 parts by mass of granulated particles thus obtained and 800 parts by mass of hydrous polymer obtained by pulverization with use of a meat chopper (REMACOM HL-3225N) in Reference Example 1 were spread on a 50-mesh metal net (having mesh size of 300 pm). Thereafter, the same operations as in Reference Example 1 were carried out. In this manner, a particulate water absorbent resin (7) was obtained. The water absorbent resin (7) thus obtained had CRC of 32.5 g/g and a soluble component of 8.9 wt%.

(Example 15)

The same operations as in Example 1 were carried out except that the water absorbent resin (1) used in Example 1 was changed to the water absorbent resin (7) and that the heat treatment time was changed to 22 minutes. In this manner, a water absorbing agent (15) was obtained. Physical properties of the water absorbing agent (15) thus obtained is shown in Table 2.

(Comparative Example 12)

The same operations as in Comparative Example 2 were carried out except that the water absorbent resin (1) used in Comparative Example 2 was changed to the water absorbent resin (7) and that the heat treatment time was changed to 40 minutes. In this manner, a comparison-use water absorbing agent (12) was obtained. Physical properties of the comparison-use water absorbing agent (12) thus obtained is shown in Table 2.

(Example 16)

The same operations as in Example 2 were carried out except that the dew point for 3 minutes after the heat treatment in Example 2 was changed to 80 °C, and the dew point in the following operation was changed to 40 °C. In this manner, a water absorbing agent (16) was obtained. Physical properties of the water absorbing agent (16) thus obtained are shown in Table 2.

[Table 1]

| | Dew point (°C) | Heat treatment time (min) | CRC (g/g) | FSC (g/g) | AAP (g/g) | SFC ($\times 10^{-7}$ cm$^3$·sec/g) | Residual EG (ppm) | Residual PD (ppm) | Residual BD (ppm) | Residual PG (ppm) | Moisture absorption blocking rate (%) |
|---|---|---|---|---|---|---|---|---|---|---|---|
| Water absorbing agent (1) | -5°C | 25min | 27.6 | 51 | 22.4 | 63 | 375 | — | — | 3480 | 0 |
| Water absorbing agent (2) | 40°C | 30min | 27.5 | 53 | 22.7 | 99 | 470 | — | — | 3630 | 0 |
| Water absorbing agent (3) | 40°C | 30min | 27.6 | 53 | 22.9 | 102 | 436 | — | — | 3658 | 0 |
| Water absorbing agent (4) | -5°C | 25min | 27.8 | 53 | 23.3 | 102 | — | 1243 | — | 3701 | 0 |
| Water absorbing agent (5) | -5°C | 25min | 27.6 | 52 | 23.3 | 98 | — | — | 1500 | 3744 | 0 |
| Water absorbing agent (6) | 35°C | 30min | 27.0 | — | 21.8 | 131 | — | — | — | — | — |
| Comparison-use water absorbing agent (1) | 80°C | 35min | 27.8 | 49 | 23.4 | 117 | 713 | — | — | 3763 | 4 |
| Comparison-use water absorbing agent (2) | 90°C | 40min | 27.5 | 51 | 23.5 | 110 | 756 | — | — | 4006 | 11 |
| Comparison-use water absorbing agent (3) | -5°C | 25min | 27.7 | 41 | 24.8 | 54 | — | — | — | — | 100 |
| Comparison-use water absorbing agent (4) | 40°C | 30min | 27.5 | 44 | 23.5 | 66 | — | — | — | — | 100 |
| Comparison-use surface crosslinked water absorbent resin (5) | 40°C | 60min | 31.0 | 50 | 21.7 | 13 | — | 2424 | — | 2689 | 100 |
| Comparison-use surface crosslinked water absorbent resin (6) | 80°C | 40min | 27.4 | 51 | 23.1 | 99 | 702 | — | — | 3899 | 13 |
| Comparison-use water absorbing agent (7) | 90°C | 50min | 27.9 | — | 21.3 | 83 | — | — | — | — | — |

[Table 2]

| | Water absorbing agent (7) | Water absorbing agent (8) | Water absorbing agent (9) | Water absorbing agent (10) | Water absorbing agent (11) | Water absorbing agent (12) | Water absorbing agent (13) | Water absorbing agent (14) | Water absorbing agent (15) | Water absorbing agent (16) | Comparison-use water absorbing agent (8) | Comparison-use water absorbing agent (9) | Comparison-use water absorbing agent (10) | Comparison-use water absorbing agent (11) | Comparison-use water absorbing agent (12) |
|---|---|---|---|---|---|---|---|---|---|---|---|---|---|---|---|
| Dew point (°C) | 35°C | 35°C | 35°C | 35°C | 35°C | 35°C | 35°C | 35°C | 40°C | 80°C for initial 3 min of heat treatment and 40°C for the following | 80°C | 80°C | 80°C | 80°C | 90°C |
| Heat treatment time | 45min | 35min | 40min | 40min | 30min | 40min | 45min | 35min | 22min | 30min | 40min | 50min | 55min | 45min | 40min |
| CRC (g/g) | 28.6 | 28.0 | 28.6 | 28.4 | 27.4 | 27.4 | 27.8 | 29.0 | 27.1 | 27.8 | 27 | 27.2 | 28.3 | 29.1 | 27.3 |
| FSC (g/g) | — | — | — | — | — | — | — | — | — | — | — | — | — | — | — |
| AAP (g/g) | — | — | — | — | 21.5 | 21.2 | 20 | 20.2 | 22.1 | 23.5 | 24.6 | 24.5 | 22.9 | 22 | 23.2 |
| SFC ($\times 10^7$ cm$^3$·sec/g) | 90 | 111 | 101 | 108 | 99 | 100 | 85 | 11 | 58 | 105 | 155 | 164 | 140 | 15 | 95 |

In Table 1, "Residual EG" indicates the amount of residual ethylene glycol, "Residual PD" indicates the amount of residual propylene glycol, "Residual BD" indicates the amount of residual 1,4-butanediol, and "Residual PG" indicates the amount of 1,3-propanediol. It should be noted that "Residual EG," "Residual PD," "Residual BD," and "Residual PG" are values of mass (ppm) contained in a water absorbing agent.

[Table 3]

| Particle size distribution (%) | on710μm | on600μm | on500μm | on300μm | on150μm | 150μm pass | D50 (μm) |
|---|---|---|---|---|---|---|---|
| Water absorbent resin (1) | 0.0 | 7.5 | 17.2 | 55.8 | 19.0 | 0.5 | 399 |
| Water absorbent resin (2) | 0.0 | 7.1 | 16.3 | 52.8 | 18.5 | 5.3 | 386 |
| Water absorbing agent (1) | 0.0 | 7.8 | 17.5 | 55.7 | 18.6 | 0.4 | 401 |
| Water absorbing agent (2) | 0.0 | 8.2 | 17.6 | 54.3 | 19.3 | 0.6 | 400 |
| Water absorbing agent (3) | 0.0 | 7.4 | 16.5 | 54.3 | 21.0 | 0.8 | 392 |
| Comparison-use water absorbing agent (1) | 0.0 | 7.5 | 16.9 | 55.4 | 19.4 | 0.8 | 397 |
| Comparison-use water absorbing agent (2) | 0.0 | 7.2 | 16.9 | 55.2 | 19.8 | 0.9 | 395 |
| Comparison-use surface crosslinked water absorbent resin (5) | 0.0 | 6.8 | 15.9 | 52.6 | 19.0 | 5.7 | 383 |
| Comparison-use surface crosslinked water absorbent resin (6) | 0.0 | 6.9 | 15.9 | 53.5 | 22.4 | 1.3 | 385 |

In Table 3, "on 710μm" indicates mass% of a water absorbent resin or water absorbing agent remaining on the sieve having mesh size of 710 μm. The same applies to "on 600μm," "on 500μm," "on 300μm," "on 150μm." Further, "150μm pass" indicates mass% of a water absorbent resin or water absorbing agent passing through the sieve having mesh size of 150 μm. "D50 (μm)" indicates the mass average particle diameter.

(Conclusion)

Examples 1 and 2 and Comparative Examples 1 and 2 were carried out for comparison between the same water absorbent resins (1) (CRC of 33 g/g) which were heated at 196 °C for surface crosslinking with use of the same surface crosslinking agent until their water absorption capacities reach the same value (CRC of approximately 27.5 g/g).
Examples 1 and 2 (dew point of lower than 45 °C) showed "reaction time of 25 to 35 minutes, residual ethylene glycol of 375 to 470 ppm, and moisture absorption blocking of 0 %". On the contrary, Comparative Examples 1 and 2 (dew point of not lower than 45 °C) showed "reaction time of 35 to 40 minutes, residual ethylene glycol of 713 to 756 ppm, and moisture absorption blocking of 4 to 11 %". As a result of comparison between Examples 1 and 2 and Comparative Examples 1 and 2, it is apparent that controlling the dew point to be low at lower than 45 °C increases productivity up to approximately 1.6 times (reaction time: 40 minutes/25 minutes) and improves properties regarding a residual ethylene glycol and moisture absorption blocking property. This result was also confirmed in the continuous surface crosslinking performed by the paddle drier in Example 3.
Similarly, comparisons between Example 11 and Comparative Example 8, between Example 12 and Comparative Example 9, between Example 13 and Comparative Example 10, and between Example 14 and Comparative Example 11 show that a reaction time taken until their water absorption capacities reach the same value is shortened.
Comparison between Example 15 and Comparative Example 12 shows that in a case where a water absorbent resin was used in which granulated particles containing a fine powder after surface treating are mixed, controlling the dew point to be low at lower than 45 °C further increases productivity to approximately 1.8 times (reaction time: 40 minutes/22 minutes).
Comparison between Example 16 and Example 2 shows that even when the dew point for initial 3 minutes during the heat treatment is 80 °C, it does not affect the reaction time as long as the dew point after the initial 3 minutes is lower than 45 °C.
Conditions in Comparative Examples 3 and 4 show that liquid permeability is not so high and that a moisture absorption blocking property is low. Comparison between Examples 1 and 2 and Comparative Examples 3 and 4 shows that a particular additive which is not disclosed in Patent Literature 36 or Patent Literature 38 is important for liquid permeability and moisture absorption blocking property.

Comparative Example 5, which carried out surface crosslinking in accordance with Example 7 of Patent Literature 36, shows that moisture absorption blocking property is 100 % and that liquid permeability is low.

Comparisons of Examples 3, 4, and 5 show that an alkylene carbonate (particularly, 2-oxo-1,3-dioxolane) as a surface crosslinking agent works effectively because it controls the dew point to be low, generates less residual surface crosslinking agent than residual surface crosslinking agents generated in the other methods of the present invention in which particular compounds are added.

As is apparent from comparison between Examples 2 and 3 and Comparative Example 6, it is shown that a large-scale continuous production (e.g. not less than 100 kg/hr, particularly not less than 1 ton/hr) through surface treating performed by still stand heating (comparison between Examples 2 and 3) increases mechanical damage to powder due to the use of a continuous stirring paddle, increases mechanical damage due to an increase in reaction time (Example 3 and Comparative Example 6), and thus increases a fine powder of a water absorbent resin. The method of the present invention enables not only shortening of a reaction time (enhancement of productivity) but also reduction of a fine powder increased by mechanical damage during the continuous production.

(Comparison to Conventional Technique)

The present invention employs a method which is not disclosed in Patent Literatures 1 to 36, thereby not only obtaining a water absorbing agent having a high physical property but also enhancing productivity and reducing a residual surface crosslinking agent (and increasing Anti-Caking property). The present invention employs a particular additive which is not disclosed in Patent Literatures 36 to 39, particularly in Patent Literature 36, thereby increasing liquid permeability and moisture absorption blocking property. The present invention employs a method which is not disclosed in Patent Literatures 40 to 44, thereby reducing a residual surface crosslinking agent, particularly ethylene glycol derived from an alkylene carbonate (particularly 2-oxo-1,3-dioxolane).

The present invention is not limited to the descriptions of the Embodiments, but can be altered within the scope of the claims. An embodiment derived from a proper combination of technical means disclosed in different embodiments is also encompassed in the technical scope of the present invention. Moreover, a novel technical feature can be created by a combination of technical means disclosed in these embodiments.

(Conclusion of the present invention)

In order to solve the above problems, the inventors of the present invention made a diligent study and accomplished the present invention by finding that in a method for producing a water absorbing agent, in which a polyacrylic acid (salt)-based water absorbent resin obtained by polymerization of a hydrophilic unsaturated monomer is subjected to a surface crosslinking agent addition step of adding a surface crosslinking agent and/or a surface crosslinking agent solution and a surface crosslinking step, wherein (i) in the surface crosslinking step performed after the surface crosslinking agent addition step, a maximum temperature in an atmosphere within a heating section of a heating apparatus used in the surface crosslinking step is in a range from 100 °C to 300 °C, and a minimum dew point in the atmosphere is lower than 45 °C, and wherein (ii) a liquid permeability enhancer addition step of adding a liquid permeability enhancer is performed simultaneously with the surface crosslinking agent addition step and/or after the surface crosslinking step, whereby a water absorbing agent having a high liquid permeability and, an excellent antiblocking property (Anti-Caking property) after moisture absorption, as an additional effect, is obtained with high productivity. Furthermore, the inventors of the present invention also found surprisingly that (a) particles obtained by drying and pulverization of fine particles removed by the classification step and then granulated are recycled for a water absorbent resin to be subjected to the surface crosslinking step, thereby further enhancing productivity, and that (b) containing fine particles which are removed from the water absorbing agent undergoing a second classification step performed after the surface crosslinking step provides a noticeable enhancement in productivity.

That is, the present invention is a method for producing a polyacrylic acid (salt)-based water absorbing agent, including:

a surface crosslinking agent addition step of adding a surface crosslinking agent to a water absorbent resin powder;
a surface crosslinking step; and
a liquid permeability enhancer addition step of adding a liquid permeability enhancer that is selected from insoluble fine particulate compounds and polyvalent cationic compounds, the liquid permeability enhancer addition step being performed simultaneously with the surface crosslinking agent addition step and/or after the surface crosslinking step, wherein a maximum temperature in an atmosphere within a heating section of a heating apparatus used in the surface crosslinking step is in a range from 100 °C to 300 °C, and a minimum dew point in the atmosphere is lower than 45 °C, and (1) an airflow rate in the heating apparatus is more than 0 but not more than 10,000 Nm3 /hr or (2) an airflow in the heating apparatus has a ratio of gas relative to an amount of the water absorbent resin powder of not more than 3000 Nm3 /ton, and the dew point is adjusted by the airflow using steam, dry air, nitrogen, helium, argon, dried air, and/or water vapor generated from water contained in the water absorbent resin powder.

In the surface crosslinking step, the water absorbent resin powder is heated so that the maximum temperature of the water absorbent resin powder is preferably in a range from 175 °C to 230 °C, more preferably in a range from 180 °C to 300 °C, still more preferably in a range from 180 °C to 250 °C, and particularly preferably in a range from 180 °C to 230 °C. A temperature of lower than 175 °C during the heating is not preferable because it may form insufficient covalent bonds for surface crosslinking. A temperature of higher than 300 °C during the heating is not preferable because it may deteriorate a resultant water absorbent resin.

Further, it is preferable that the method for producing a polyacrylic acid (salt)-based water absorbing agent further includes a fine powder recycling step.

The liquid permeability enhancer addition step is carried out at least once. The liquid permeability enhancer addition step may be carried out during the surface crosslinking agent addition step or may be carried out after the surface crosslinking step. Alternatively, the liquid permeability enhancer addition step may be carried out twice, i.e. during the surface crosslinking agent addition step and after the surface crosslinking step.

The polyacrylic acid (salt)-based water absorbent resin to be subjected to the surface crosslinking agent addition step has a temperature preferably in a range from 30 °C to 100 °C, more preferably in a range from 35 °C to 80 °C, and still more preferably in a range from 40 °C to 70 °C. A temperature of lower than 30 °C is not preferable because it may cause moisture absorption of the water absorbent resin and decrease in fluidity, thus causing a difficulty in handling in production. A temperature of higher than 100 °C is not preferable because it causes sudden evaporation of an added surface crosslinking agent solution, thus preventing a surface crosslinking agent from being evenly added.

A minimum dew point in the atmosphere where the heat treatment is performed is lower than 45 °C, and a lower limit of the dew point is preferably not lower than -30 °C, more preferably not lower than -10 °C, and still more preferably not lower than 0 °C.

Further, a time for the heat treatment is in a range from 1 to 120 minutes and more preferably in a range from 5 to 60 minutes.

The liquid permeability enhancer used in the liquid permeability enhancer addition step is selected from water-insoluble inorganic fine particles, a cationic polymer compound, a water-soluble polyvalent metal cation-containing compound containing a bivalent or higher metal cation, water-soluble polysiloxane, and an amine compound containing oxyalkylene group whose carbon number is 8 or more.

The liquid permeability enhancer is added preferably in an amount in a range from 0.01 to 5 parts by mass, more preferably in an amount in a range from 0.01 to 2 parts by mass, and still more preferably in an amount in a range from 0.01 to 1 part by mass, relative to 100 parts by mass of a water absorbent resin.

In order to control a dew point in the system where the above surface crosslinking process is performed, it is preferable that a gas having a temperature of not lower than 30 °C but lower than 100 °C and having a dew point of not lower than -100 °C but not higher than 30 °C, is introduced into the heating section. Further, it is more preferable that the gas to be introduced is selected from dry air, nitrogen, helium, argon, carbon dioxide, and steam.

It is preferable that a surface crosslinking agent solution used in the surface crosslinking treatment contains an organic surface crosslinking agent that forms, when subjected to heat treatment, a covalent bond with a carboxyl group which exists in vicinity of a surface of a water absorbent resin. The organic surface crosslinking agent is preferably at least one substance selected from a polyhydric alcohol, an oxazoline compound, an epoxy compound, an alkylene carbonate compound, and an oxetane compound. Particularly, it is preferable that the organic surface crosslinking agent is an alkylene carbonate compound or contains an alkylene carbonate compound and at least one other type of compound other than the alkylene carbonate compound. It is more preferable that the organic surface crosslinking agent is a composite organic surface crosslinking agent containing at least two types of compounds.

Further, the organic surface crosslinking agent is added preferably in an amount in a range from 0.01 to 10 parts by mass and more preferably in an amount in a range from 0.1 to 5 parts by mass, relative to 100 parts by mass of the water absorbent resin.

The surface crosslinking agent solution may contain water in an amount preferably in a range from 0 to 10 parts by mass and more preferably in a range from 1 to 5 parts by mass, relative to 100 parts by mass of the water absorbent resin.

It is preferable that a Coriolis mass flowmeter is used to weigh the surface crosslinking agent and/or the surface crosslinking agent solution. The Coriolis mass flowmeter is less likely to cause a measurement error derived from a temperature, etc. of an object to be measured and enables an accurate weighing.

It is preferable that the surface crosslinking agent used in the surface crosslinking agent addition step is the organic surface crosslinking agent solution adjusted so as to have moisture content in a range from 1 to 10 parts by mass relative to 100 parts by mass of water absorbent resin powder.

A water absorbing agent of the present invention preferably contains fewer fine powder of less than 150 μm in diameter, and an amount of the fine powder of less than 150 μm in diameter is preferably less than 5 mass%. Further, a mass average particle diameter of the water absorbing agent is preferably not less than 200 μm but not more than 600 μm, more preferably in a range from 200 μm to 550 μm, and still more preferably in a range from 250 μm to 500 μm, and most preferably in a range from 350 μm to 450 μm.

The particle diameter and particle size distribution of the water absorbing agent is preferably applied similarly to a water absorbent resin to be subjected to the surface crosslinking agent addition step. For this reason, it is preferable that a pulverizing step and/or a classification step is performed before the surface crosslinking agent addition step.

In order to obtain a water absorbing agent having higher physical properties, it is preferable to perform a surfactant addition step, and the surfactant addition step may be performed simultaneously with the surface crosslinking agent solution addition step and/or after the surface crosslinking step. It should be noted that "simultaneously with the surface crosslinking agent solution addition step" means the form of adding a surface crosslinking agent and a surfactant separately at the same time and/or the form of adding a mixture of the surface crosslinking agent and the surfactant.

Further, in order to enhance the efficiency of heating and perform an even heat treatment, it is preferable to use an apparatus including a mechanism for continuously stirring and/or fluidizing an object to be heated. A stirring and/or a fluidizing method is preferably a channel stirring method, a method of a screw type, a method of a rotary type, a method of a disc type, a method of a kneading type, a method of a fluidized-bed type, and the like. It is more preferable that a stirring method using stirring blades (paddles) and a stirring method based on movement of a heat transfer surface itself by means of a rotary retort furnace. It should be noted that the stirring and/or fluidizing mechanism used for the purpose of performing an even heat treatment need not to be used in a case where the amount of object to be dried is small, for example, in a case where a dried product has a thickness of less than 1 cm.

Industrial Applicability

According to a production method of the present invention, it is possible to obtain a water absorbing agent having high physical properties (particularly high liquid permeability and high Anti-Caking property) and high productivity, and containing a residual surface crosslinking agent in a low amount. Thus, it is possible to supply a large amount of water absorbing agent at low cost and with a high degree of safety, for use in sanitary materials such as disposable diapers.

**Claims**

1. A method for producing a polyacrylic acid (salt)-based water absorbing agent, comprising:

   a surface crosslinking agent addition step of adding a surface crosslinking agent to a water absorbent resin powder;
   a surface crosslinking step; and
   a liquid permeability enhancer addition step of adding a liquid permeability enhancer that is selected from insoluble fine particulate compounds and polyvalent cationic compounds, the liquid permeability enhancer addition step being performed simultaneously with the surface crosslinking agent addition step and/or after the surface crosslinking step,
   wherein a maximum temperature in an atmosphere within a heating section of a heating apparatus used in the surface crosslinking step is in a range from 100 °C to 300 °C, and a minimum dew point in the atmosphere is lower than 45 °C, wherein the temperature and dew point are values measured in an upper space plumb to a water absorbent resin powder being heated in the heating section, and

   (1) an airflow rate in the heating apparatus is more than 0 but not more than 10,000 Nm$^3$/hr or (2) an airflow in the heating apparatus has a ratio of gas relative to an amount of the water absorbent resin powder of not more than 3000 Nm$^3$/ton, and the dew point is adjusted by the airflow using steam, dry air, nitrogen, helium, argon, dried air, and/or water vapor generated from water contained in the water absorbent resin powder.

2. The method according to claim 1, wherein
   the liquid permeability enhancer is at least one substance selected from a polyvalent metal cation-containing compound, a water-insoluble inorganic fine particles, and a cationic polymer compound, and is added in an amount in a range from 0.01 to 5 parts by mass, relative to 100 parts by mass of the water absorbent resin powder.

3. The method according to claim 2, wherein

the liquid permeability enhancer is an aqueous solution containing the polyvalent metal cation-containing compound, and the aqueous solution is heated.

4. The method according to any one of the claims 1 to 3, wherein
a gas having a temperature of not lower than 30 °C but lower than 100 °C and having a dew point of not lower than -100 °C but not higher than 30 °C is introduced into the heating section.

5. The method according to claim 4, wherein
the gas is one selected from dry air, nitrogen, helium, argon, carbon dioxide, and steam.

6. The method according to any one of claims 1 to 5, wherein
the surface crosslinking agent is added in the surface crosslinking agent addition step to a water absorbent resin powder,
the water absorbent resin powder to be subjected to the surface crosslinking agent addition step has a temperature in a range from 30 °C to 100 °C, and
the surface crosslinking step heats the water absorbent resin powder so that a maximum temperature of the water absorbent resin powder is in a range from 175 °C to 230 °C.

7. The method according to any one of claims 1 to 6, wherein
a time for heat treatment in the surface crosslinking step is in a range from 5 to 60 minutes.

8. The method according to any one of claims 1 to 7, wherein
the surface crosslinking agent is added in the surface crosslinking agent addition step to a water absorbent resin powder,
the surface crosslinking agent used in the surface crosslinking agent addition step is an organic surface crosslinking agent, and the organic surface crosslinking agent is added in an amount in a range from 0.1 to 10 parts by mass relative to 100 parts by mass of the water absorbent resin powder.

9. The method according to any one of claims 1 to 8, wherein
the surface crosslinking agent contains at least one compound selected from a polyhydric alcohol compound and/or an amino alcohol, an alkylene carbonate, an oxazolidinone compound, and an epoxy compound.

10. The method according to any one of claims 1 to 9, wherein
the surface crosslinking agent contains an alkylene carbonate or an aqueous solution of alkylene carbonate, the alkylene carbonate or the aqueous solution of alkylene carbonate being heated.

11. The method according to any one of claims 1 to 10, wherein
the surface crosslinking agent added in the surface crosslinking agent addition step is a composite organic surface crosslinking agent containing a plurality of organic surface crosslinking agents.

12. The method according to any one of claims 1 to 11, wherein
in preparation of the composite organic surface crosslinking agent, a mixture ratio is controlled by a Coriolis mass flowmeter.

13. The method according to any one of claims 1 to 12, wherein
the surface crosslinking agent is added in the surface crosslinking agent addition step to a water absorbent resin powder, and
the surface crosslinking agent used in the surface crosslinking agent addition step is a surface crosslinking agent solution adjusted so as to have moisture content in a range from 1 to 10 parts by mass relative to 100 parts by mass of the water absorbent resin powder.

14. The method according to any one of claims 1 to 13, further comprising:
a fine powder recycling step and/or a surfactant addition step.

15. A water absorbing agent obtainable by the method according to any one of claims 1 to 14.

**Patentansprüche**

1. Verfahren zur Herstellung eines Wasserabsorptionsmittels auf Polyacrylsäure(salz)basis, umfassend:

einen Oberflächenvernetzungsmittel-Hinzufügeschritt, in dem ein Oberflächenvernetzungsmittel zu einem wasserabsorbierenden Harzpulver hinzugefügt wird;
einen Oberflächenvernetzungsschritt; und
einen Flüssigpermeabilitätsverbesserungsmittel-Hinzufügeschritt, in dem ein Flüssigpermeabilitätsverbesserungsmittel, das aus unlöslichen Feinpartikel-förmigen Verbindungen und polyvalenten kationischen Verbindungen ausgewählt ist, hinzugefügt wird, wobei der Flüssigpermeabilitätsverbesserungsmittel-Hinzufügeschritt gleichzeitig mit dem Oberflächenvernetzungsmittel-Hinzufügeschritt und/oder nach dem Oberflächenvernetzungsschritt durchgeführt wird,
wobei eine Maximaltemperatur in einer Atmosphäre innerhalb eines Erwärmungsbereichs einer Erwärmungsvorrichtung, die in dem Oberflächenvernetzungsschritt verwendet wird, im Bereich von 100 °C bis 300 °C liegt, und ein Minimaltaupunkt in der Atmosphäre geringer als 45 °C ist, wobei die Temperatur und der Taupunkt Werte sind, die im Lot eines oberen Raumes (upper space plumb) zu einem in dem Erwärmungsbereich erwärmten wasserabsorbierenden Harzpulver gemessen wurden, und

(1) eine Luftflussrate in der Erwärmungsvorrichtung mehr als 0 aber nicht mehr als 10,000 Nm$^3$/Stunde beträgt oder (2) eine Luftflussrate in der Erwärmungsvorrichtung ein Verhältnis von Gas relativ zu einer Menge des wasserabsorbierenden Harzpulvers von nicht mehr als 3000 Nm$^3$/Tonne aufweist, und der Taupunkt eingestellt wird durch den Luftfluss unter Verwendung von Dampf, trockener Luft, Stickstoff, Helium, Argon, getrockneter Luft und/oder Wasserdampf, der aus Wasser, das in dem wasserabsorbierenden Harzpulver enthalten ist, generiert ist.

2. Verfahren gemäß Anspruch 1, bei dem
das Flüssigpermeabilitätsverbesserungsmittel mindestens eine Substanz, ausgewählt aus einer polyvalente Metallkationen-haltigen Verbindung, wasserunlöslichen anorganischen Feinpartikeln und einer kationischen Polymerverbindung, ist und in einer Menge im Bereich von 0,01 bis 5 Massenteilen, bezogen auf 100 Massenteile des wasserabsorbierenden Harzpulvers, hinzugefügt wird.

3. Verfahren gemäß Anspruch 2, bei dem
das Flüssigpermeabilitätsverbesserungsmittel eine wässrige Lösung ist, die die polyvalente Metallkationen-haltigen Verbindung enthält, und wobei die wässrige Lösung erwärmt wird.

4. Verfahren gemäß mindestens einem der Ansprüche 1 bis 3, bei dem
ein Gas mit einer Temperatur von nicht geringer als 30 °C aber geringer als 100 °C und mit einem Taupunkt von nicht geringer als -100 °C aber nicht höher als 30 °C in den Erwärmungsbereich eingeleitet wird.

5. Verfahren gemäß Anspruch 4, bei dem
das Gas eines, ausgewählt aus trockener Luft, Stickstoff, Helium, Argon, Kohlenstoffdioxid und Dampf, ist.

6. Verfahren gemäß mindestens einem der Ansprüche 1 bis 5, bei dem
das Oberflächenvernetzungsmittel in dem Oberflächenvernetzungsmittel-Hinzufügeschritt zu einem wasserabsorbierenden Harzpulver hinzugefügt wird,
das wasserabsorbierende Harzpulver, welches dem Oberflächenvernetzungsmittel-Hinzufügeschritt ausgesetzt wird, eine Temperatur im Bereich von 30 °C bis 100 °C aufweist, und
der Oberflächenvernetzungsschritt das wasserabsorbierende Harzpulver so erwärmt, dass eine Maximaltemperatur des wasserabsorbierenden Harzpulvers im Bereich von 175 °C bis 230 °C liegt.

7. Verfahren gemäß mindestens einem der Ansprüche 1 bis 6, bei dem
eine Zeit der Wärmebehandlung in dem Oberflächenvernetzungsschritt im Bereich von 5 bis 60 Minuten liegt.

8. Verfahren gemäß mindestens einem der Ansprüche 1 bis 7, bei dem
das Oberflächenvernetzungsmittel in dem Oberflächenvernetzungsmittel-Hinzufügeschritt zu einem wasserabsorbierenden Harzpulver hinzugefügt wird,
das in dem Oberflächenvernetzungsschritt verwendete Oberflächenvernetzungsmittel ein organisches Oberflächenvernetzungsmittel ist, und das organische Oberflächenvernetzungsmittel in einer Menge im Bereich von 0,1 bis 10 Massenteilen, bezogen auf 100 Massenteile des wasserabsorbierenden Harzpulvers, hinzugefügt wird.

9. Verfahren gemäß mindestens einem der Ansprüche 1 bis 8, bei dem
   das Oberflächenvernetzungsmittel mindestens eine Verbindung, ausgewählt aus einer mehrwertigen Alkoholverbindung und/oder einem Aminoalkohol, einem Alkylencarbonat, einer Oxazolidinonverbindung und einer Epoxidverbindung, enthält.

10. Verfahren gemäß mindestens einem der Ansprüche 1 bis 9, bei dem
    das Oberflächenvernetzungsmittel ein Alkylencarbonat oder eine wässrige Lösung eines Alkylencarbonats enthält, wobei das Alkylencarbonat oder die wässrige Lösung des Alkylencarbonats erwärmt wird.

11. Verfahren gemäß mindestens einem der Ansprüche 1 bis 10, bei dem
    das Oberflächenvernetzungsmittel, das in dem Oberflächenvernetzungsmittel-Hinzufügeschritt hinzugefügt wird, ein organisches Oberflächenvernetzungsverbundmittel ist, das mehrere organische Oberflächenvernetzungsmittel enthält.

12. Verfahren gemäß mindestens einem der Ansprüche 1 bis 11, bei dem
    in der Herstellung des organischen Oberflächenvernetzungsverbundmittels ein Mischungsverhältnis durch ein Coriolis Massendurchflussmesser kontrolliert wird.

13. Verfahren gemäß mindestens einem der Ansprüche 1 bis 12, bei dem
    das Oberflächenvernetzungsmittel in dem Oberflächenvernetzungsmittel-Hinzufügeschritt zu einem wasserabsorbierenden Harzpulver hinzugefügt wird, und
    das Oberflächenvernetzungsmittel, das in dem Oberflächenvernetzungsmittel-Hinzufügeschritt verwendet wird, eine Oberflächenvernetzungsmittellösung ist, die so eingestellt ist, dass sie einen Feuchtigkeitsgehalt im Bereich von 1 bis 10 Massenteilen, bezogen auf 100 Massenteile des wasserabsorbierenden Harzpulvers, aufweist.

14. Verfahren gemäß mindestens einem der Ansprüche 1 bis 13, ferner umfassend:
    einen Feinpulverrecyclingschritt und/oder einen Tensid-Hinzufügeschritt.

15. Wasserabsorptionsmittel, das durch das Verfahren gemäß mindestens einem der Ansprüche 1 bis 14 erhältlich ist.

**Revendications**

1. Procédé de production d'un agent absorbant l'eau à base d'acide polyacrylique (sel), comprenant :

   une étape d'addition d'agent de réticulation de surface consistant à ajouter un agent de réticulation de surface à une poudre de résine absorbant l'eau ;
   une étape de réticulation de surface ; et
   une étape d'addition d'agent d'amélioration de perméabilité aux liquides consistant à ajouter un agent d'amélioration de perméabilité aux liquides qui est choisi parmi des composés insolubles en fines particules et des composés cationiques polyvalents, l'étape d'addition d'agent d'amélioration de perméabilité aux liquides étant effectuée simultanément avec l'étape d'addition d'agent de réticulation de surface et/ou après l'étape de réticulation de surface,
   dans lequel une température maximum dans une atmosphère dans une section de chauffage d'un appareil de chauffage utilisé dans l'étape de réticulation de surface est dans une plage allant de 100 °C à 300°C, et un point de rosée minimum dans l'atmosphère est inférieur à 45 °C,
   dans lequel la température et le point de rosée sont des valeurs mesurées dans un espace supérieur à l'aplomb d'une poudre de résine absorbant l'eau qui est chauffée dans la section de chauffage, et

   (1) un débit d'écoulement d'air dans l'appareil de chauffage est supérieur à 0 mais pas supérieur à 10 000 Nm$^3$/h ou (2) un écoulement d'air dans l'appareil de chauffage a un rapport de gaz par rapport à une quantité de la poudre de résine absorbant l'eau non supérieure à 3 000 Nm$^3$/tonne, et le point de rosée est ajusté par l'écoulement d'air en utilisant de la vapeur, de l'air sec, de l'azote, de l'hélium, de l'argon, de l'air séché et/ou de la vapeur d'eau générée à partir de l'eau contenue dans la poudre de résine absorbant l'eau.

2. Procédé selon la revendication 1, dans lequel

l'agent d'amélioration de perméabilité aux liquides est au moins une substance choisie parmi un composé contenant un cation de métal polyvalent, des particules fines inorganiques insolubles dans l'eau et un composé polymère cationique, et est ajouté en une quantité dans une plage allant de 0,01 à 5 parties en masse, par rapport à 100 parties en masse de la poudre de résine absorbant l'eau.

3. Procédé selon la revendication 2, dans lequel
l'agent d'amélioration de perméabilité aux liquides est une solution aqueuse contenant le composé contenant un cation de métal polyvalent, et la solution aqueuse est chauffée.

4. Procédé selon l'une quelconque des revendications 1 à 3, dans lequel
un gaz présentant une température pas inférieure à 30 °C mais inférieure à 100 °C et dont le point de rosée n'est pas inférieur à -100 °C mais pas supérieur à 30 °C est introduit dans la section de chauffage.

5. Procédé selon la revendication 4, dans lequel
le gaz est choisi parmi l'air sec, l'azote, l'hélium, l'argon, le dioxyde de carbone et la vapeur.

6. Procédé selon l'une quelconque des revendications 1 à 5, dans lequel
l'agent de réticulation de surface est ajouté dans l'étape d'addition d'agent de réticulation de surface à une poudre de résine absorbant l'eau,
la poudre de résine absorbant l'eau devant être soumise à l'étape d'addition d'agent de réticulation de surface présente une température dans une plage allant de 30 °C à 100 °C, et
l'étape de réticulation de surface chauffe la poudre de résine absorbant l'eau de sorte qu'une température maximum de la poudre de résine absorbant l'eau se situe dans une plage allant de 175 °C à 230 °C.

7. Procédé selon l'une quelconque des revendications 1 à 6, dans lequel
une durée du traitement thermique dans l'étape de réticulation de surface est dans une plage allant de 5 à 60 minutes.

8. Procédé selon l'une quelconque des revendications 1 à 7, dans lequel
l'agent de réticulation de surface est ajouté dans l'étape d'addition d'agent de réticulation de surface à une poudre de résine absorbant l'eau,
l'agent de réticulation de surface utilisé dans l'étape d'addition d'agent de réticulation de surface est un agent de réticulation de surface organique, et l'agent de réticulation de surface organique est ajouté en une quantité dans une plage allant de 0,1 à 10 parties en masse par rapport à 100 parties en masse de la poudre de résine absorbant l'eau.

9. Procédé selon l'une quelconque des revendications 1 à 8, dans lequel
l'agent de réticulation de surface contient au moins un composé choisi parmi un composé d'alcool polyhydrique et/ou un aminoalcool, un carbonate d'alkylène, un composé d'oxazolidinone, et un composé époxy.

10. Procédé selon l'une quelconque des revendications 1 à 9, dans lequel
l'agent de réticulation de surface contient un carbonate d'alkylène ou une solution aqueuse de carbonate d'alkylène, le carbonate d'alkylène ou la solution aqueuse de carbonate d'alkylène étant chauffé(e).

11. Procédé selon l'une quelconque des revendications 1 à 10, dans lequel
l'agent de réticulation de surface ajouté dans l'étape d'addition d'agent de réticulation de surface est un agent de réticulation de surface organique composite contenant une pluralité d'agents de réticulation de surface organiques.

12. Procédé selon l'une quelconque des revendications 1 à 11, dans lequel
lors de la préparation de l'agent de réticulation de surface organique composite, un rapport de mélange est commandé par un débitmètre massique de Coriolis.

13. Procédé selon l'une quelconque des revendications 1 à 12, dans lequel
l'agent de réticulation de surface est ajouté dans l'étape d'addition d'agent de réticulation de surface à une poudre de résine absorbant l'eau, et
l'agent de réticulation de surface utilisé dans l'étape d'addition d'agent de réticulation de surface est une solution d'agent de réticulation de surface ajustée de manière à avoir une teneur en humidité dans une plage allant de 1 à 10 parties en masse par rapport à 100 parties en masse de la poudre de résine absorbant l'eau.

**14.** Procédé selon l'une quelconque des revendications 1 à 13, comprenant en outre :
une étape de recyclage de poudre fine et/ou une étape d'addition de tensioactif.

**15.** Agent absorbant l'eau pouvant être obtenu par le procédé selon l'une quelconque des revendications 1 à 14.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9526209 A
- US 6297319 B
- US 6372852 B
- US 6265488 B
- US 6809158 B
- US 4734478 A
- US 4755562 A
- US 4824901 A
- US 6239230 B
- US 6559239 B
- US 6472478 B
- US 6657015 B
- US 5672633 A
- EP 0940149 A
- WO 2006033477 A
- US 7201941 B
- US 4783510 A
- EP 1824910 A
- WO 2002100451 A
- US 5610208 A
- WO 92000108 A
- WO 9849221 A
- WO 0053644 A
- WO 0053664 A
- WO 01074913 A
- WO 2002020068 A
- WO 2002022717 A
- WO 2005080479 A
- WO 2007065834 A
- WO 2008092842 A
- WO 2008092843 A
- WO 2008110524 A
- WO 2009080611 A
- JP 4046617 A
- WO 0046260 A
- EP 1191051 A
- WO 2011117263 A
- WO 09125849 A
- KR 20110049072 A
- WO 2006042704 A
- EP 0668080 A
- WO 970003114 A
- US 7378453 B
- WO 2012102406 A
- WO 2012102407 A
- US 2006204755 A
- US 5669894 A
- WO 2005016393 A
- WO 2008096713 A
- US 4625001 A
- US 4873299 A
- US 4286082 A
- US 4973632 A
- US 4985518 A
- US 5124416 A
- US 5250640 A
- US 5264495 A
- US 5145906 A
- US 5380808 A
- EP 0811636 A
- EP 0955086 A
- EP 0922717 A
- US 4093776 A
- US 4367323 A
- US 4446261 A
- US 4683274 A
- US 5244735 A
- JP 1318021 A
- WO 2011078298 A
- US 4893999 A
- US 6241928 B
- US 2005215734
- WO 2008114847 A
- US 6987151 B
- US 6710141 B
- WO 2008114848 A
- JP 2000063527 A
- WO 200469915 A
- US 92001008 W
- US 92020723 PCT
- US 2007074167 W
- US 2009109563 PCT
- US 2009153196 A
- US 2010006937 PCT
- US 6228930 B
- US 7473739 B
- WO 97017397 A
- US 6107358 A
- WO 2004096304 A
- US 5382610 A
- US 7098284 B
- WO 2009110645 A
- WO 2009041731 A
- WO 2009041727 A
- WO 2009093708 A
- WO 2008108343 A
- US 5562646 A
- WO 2009125849 A

• EP 119051 A

**Non-patent literature cited in the description**

• *Modern Superabsorbent Polymer Technology,* 1998